# EUROPÄISCHE PATENTANMELDUNG

(11) **EP 2 108 695 A1**
(43) Veröffentlichungstag der Anmeldung: **14.10.2009**
(21) Anmeldenummer: 08450052.9
(22) Anmeldetag: 08.04.2008
(51) Int. Cl.: C12N 9/64

(54) **Ace2 Polypeptid**

(71) Anmelder: Apeiron Biologics Forschungs- und Entwicklungsgesellschaft M.B.H., 1230 Wien (AT)
(72) Erfinder: Weik, Robert, 2724 Hohe Wand-Stollhof (AT)
(74) Vertreter: Sonn & Partner Patentanwälte

(57) **Zusammenfassung**

Die vorliegende Erfindung betrifft rekombinantes ACE2 Polypeptid welches glykosiliert ist, wobei die Glycogruppen eines ACE2 Polypeptid Monomers in Summe mindestens 14 Sialylsäure-Reste aufweisen und das ACE2 Polypetid als Dimer vorliegt.

SEQUENCE LISTING

<110>  Apeiron Biologics Forschungs- und Entwicklungs GmbH

<120>  ACE2 Polypeptid

<130>  r51676

<160>  1

<170>  PatentIn version 3.4

<210>  1
<211>  740
<212>  PRT
<213>  homo sapiens

<400>  1

Met Ser Ser Ser Ser Trp Leu Leu Leu Ser Leu Val Ala Val Thr Ala
1               5                   10                  15

Ala Gln Ser Thr Ile Glu Glu Gln Ala Lys Thr Phe Leu Asp Lys Phe
            20                  25                  30

Asn His Glu Ala Glu Asp Leu Phe Tyr Gln Ser Ser Leu Ala Ser Trp
        35                  40                  45

Asn Tyr Asn Thr Asn Ile Thr Glu Glu Asn Val Gln Asn Met Asn Asn
    50                  55                  60

Ala Gly Asp Lys Trp Ser Ala Phe Leu Lys Glu Gln Ser Thr Leu Ala
65                  70                  75                  80

Gln Met Tyr Pro Leu Gln Glu Ile Gln Asn Leu Thr Val Lys Leu Gln
                85                  90                  95

Leu Gln Ala Leu Gln Gln Asn Gly Ser Ser Val Leu Ser Glu Asp Lys
            100                 105                 110

Ser Lys Arg Leu Asn Thr Ile Leu Asn Thr Met Ser Thr Ile Tyr Ser
        115                 120                 125

Thr Gly Lys Val Cys Asn Pro Asp Asn Pro Gln Glu Cys Leu Leu Leu
    130                 135                 140

Glu Pro Gly Leu Asn Glu Ile Met Ala Asn Ser Leu Asp Tyr Asn Glu
145                 150                 155                 160

Arg Leu Trp Ala Trp Glu Ser Trp Arg Ser Glu Val Gly Lys Gln Leu
                165                 170                 175

Arg Pro Leu Tyr Glu Glu Tyr Val Val Leu Lys Asn Glu Met Ala Arg
            180                 185                 190

Ala Asn His Tyr Glu Asp Tyr Gly Asp Tyr Trp Arg Gly Asp Tyr Glu
        195                 200                 205

Val Asn Gly Val Asp Gly Tyr Asp Tyr Ser Arg Gly Gln Leu Ile Glu
    210                 215                 220

Asp Val Glu His Thr Phe Glu Glu Ile Lys Pro Leu Tyr Glu His Leu
225                 230                 235                 240

His Ala Tyr Val Arg Ala Lys Leu Met Asn Ala Tyr Pro Ser Tyr Ile
                245                 250                 255

Ser Pro Ile Gly Cys Leu Pro Ala His Leu Leu Gly Asp Met Trp Gly
            260                 265                 270

Arg Phe Trp Thr Asn Leu Tyr Ser Leu Thr Val Pro Phe Gly Gln Lys
        275                 280                 285

Pro Asn Ile Asp Val Thr Asp Ala Met Val Asp Gln Ala Trp Asp Ala
    290                 295                 300

Gln Arg Ile Phe Lys Glu Ala Glu Lys Phe Phe Val Ser Val Gly Leu
305                 310                 315                 320

Pro Asn Met Thr Gln Gly Phe Trp Glu Asn Ser Met Leu Thr Asp Pro
                325                 330                 335

Gly Asn Val Gln Lys Ala Val Cys His Pro Thr Ala Trp Asp Leu Gly
            340                 345                 350

Lys Gly Asp Phe Arg Ile Leu Met Cys Thr Lys Val Thr Met Asp Asp
        355                 360                 365

Phe Leu Thr Ala His His Glu Met Gly His Ile Gln Tyr Asp Met Ala
    370                 375                 380

Tyr Ala Ala Gln Pro Phe Leu Leu Arg Asn Gly Ala Asn Glu Gly Phe
385                 390                 395                 400

His Glu Ala Val Gly Glu Ile Met Ser Leu Ser Ala Ala Thr Pro Lys
                405                 410                 415

His Leu Lys Ser Ile Gly Leu Leu Ser Pro Asp Phe Gln Glu Asp Asn
            420                 425                 430

Glu Thr Glu Ile Asn Phe Leu Leu Lys Gln Ala Leu Thr Ile Val Gly
        435                 440                 445

Thr Leu Pro Phe Thr Tyr Met Leu Glu Lys Trp Arg Trp Met Val Phe
    450                 455                 460

Lys Gly Glu Ile Pro Lys Asp Gln Trp Met Lys Lys Trp Trp Glu Met
465                 470                 475                 480

Lys Arg Glu Ile Val Gly Val Val Glu Pro Val Pro His Asp Glu Thr

                485                 490                 495

Tyr Cys Asp Pro Ala Ser Leu Phe His Val Ser Asn Asp Tyr Ser Phe
            500                 505                 510

Ile Arg Tyr Tyr Thr Arg Thr Leu Tyr Gln Phe Gln Phe Gln Glu Ala
        515                 520                 525

Leu Cys Gln Ala Ala Lys His Glu Gly Pro Leu His Lys Cys Asp Ile
    530                 535                 540

Ser Asn Ser Thr Glu Ala Gly Gln Lys Leu Phe Asn Met Leu Arg Leu
545                 550                 555                 560

Gly Lys Ser Glu Pro Trp Thr Leu Ala Leu Glu Asn Val Val Gly Ala
                565                 570                 575

Lys Asn Met Asn Val Arg Pro Leu Leu Asn Tyr Phe Glu Pro Leu Phe
            580                 585                 590

Thr Trp Leu Lys Asp Gln Asn Lys Asn Ser Phe Val Gly Trp Ser Thr
        595                 600                 605

Asp Trp Ser Pro Tyr Ala Asp Gln Ser Ile Lys Val Arg Ile Ser Leu
    610                 615                 620

Lys Ser Ala Leu Gly Asp Lys Ala Tyr Glu Trp Asn Asp Asn Glu Met
625                 630                 635                 640

Tyr Leu Phe Arg Ser Ser Val Ala Tyr Ala Met Arg Gln Tyr Phe Leu
                645                 650                 655

Lys Val Lys Asn Gln Met Ile Leu Phe Gly Glu Glu Asp Val Arg Val
            660                 665                 670

Ala Asn Leu Lys Pro Arg Ile Ser Phe Asn Phe Phe Val Thr Ala Pro
        675                 680                 685

Lys Asn Val Ser Asp Ile Ile Pro Arg Thr Glu Val Glu Lys Ala Ile
    690                 695                 700

Arg Met Ser Arg Ser Arg Ile Asn Asp Ala Phe Arg Leu Asn Asp Asn
705                 710                 715                 720

Ser Leu Glu Phe Leu Gly Ile Gln Pro Thr Leu Gly Pro Pro Asn Gln
                725                 730                 735

Pro Pro Val Ser
            740

## Beschreibung

Die vorliegende Erfindung betrifft das Gebiet der Produktion rekombinanter Proteine.

ACE2 stellt ein Schlüsselenzym des Renin-Angiotensin-Systems dar. Als Carboxypeptidase wird es membranverankert, als Rezeptor vor allem auf Lungen-, Nieren- und Herzzellen, aber auch auf Endothelzellen exprimiert und spaltet diverse Peptidsubstrate. Prominente Substratvertreter sind Apellin, Bradykinin aber ebenso Angiotensin-I, welches zu Angiotensin 1-9, oder Ang-II, welches zu Ang 1-7 gespalten werden. Ang II und Ang 1-7 sind Antagonisten des Renin-Angiotensin-Systems. ACE2 ist über die Steuerung der Peptidverhältnisse maßgeblich für die Regulation der Gefäßdicke sowie für die Endothelpermeabilität verantwortlich und beeinflusst hierbei die Homeostasis des Organismus. Die Expression von ACE2 ist zytokingesteuert und wird in diversen entzündlichen Erkrankungen herabgesetzt, was in weiterer Folge zu einer pathologischen Anreicherung von Ang II, einem der wichtigsten Substrate von ACE2, führt.

ACE2 dient zur Enzymsubstitutionstherapie zur Behandlung von ARDS oder ALI, zwei akuten Lungenerkrankungen mit welchen als Begleitsymptom ein herunterregulierter ACE2 Titer einhergeht. Zur Enzymsubstitutionstherapie wird lösliches humanes ACE2 verwendet, welches systemisch gegeben wird und binnen kürzester Zeit im gesamten Organismus zur Verfügung steht, um Titer von Ang II zu Gunsten von Ang 1-7 zu verschieben. Dies kompensiert die negativen Auswirkungen erhöhter Ang II Titer. Hierfür ist es unumgänglich ein Produkt in Händen zu haben, welches ein vernünftiges pharmakologisches Profil aufweist: Dementsprechend kennzeichnet sich eine geeignete Substanz durch eine gute Verteilung im Organismus, eine vernünftige Halbwertszeit sowie durch eine geringe Immunogenizität. Ferner muss das Produkt natürlich auch enzymatisch aktiv, gut löslich sowie auch in Lösung stabil sein und sich in ausreichender Reinheit reproduzierbar und ökonomisch darstellen lassen.

Die therapeutische Umsetzung einer effizienten Enzymsubstitutionstherapie erfordert einen Herstellungsprozess, der ökonomisch und reproduzierbar ein hochreines pharmakologisch effizientes Produkt darstellt. Der lösliche Abschnitt von ACE2 enthält 7 N-Glykosylierungsstellen. Nicht vollständig glykosyliertes ACE2 ist schlecht löslich, neigt zur Aggregation, ist potentiell immunogen und weist eine verkürzte Halbwertszeit auf. Außerdem besitzt es einen kleineren hydrodynamischen Durchmesser, welcher vor allem die Aufreinigung negativ beeinflusst. Es ist daher ein Ziel der vorliegen Erfindung hoch aktives ACE2 mit einer guten in vivo Halbwertszeit zur Verfügung zu stellen. Dieses Ziel wird durch die Gegenstände der Ansprüche erfüllt.

Die vorliegende Erfindung betrifft rekombinantes ACE2 Polypeptid, welches glykosiliert ist, wobei die Glycogruppen eines ACE2 Polypeptid Monomers in Summe mindestens 10, 11, 12, 13, 14, 15, oder mindestens 16 Sialylsäurereste aufweisen und das ACE2 Polypetid als Dimer vorliegt. Vorzugsweise enthält das Dimer zwei Zink Ionen.

Unter Sialylsäurereste werden insbesondere Reste vom N-Acetylneuraminsäure-Typ (Neu5Ac) verstanden, im speziellen an N-oder O-Glycosilierungen.

Grundsätzlich ist die Stabilität eines Therapeutikums ein sehr wichtiges Kriterium für dessen Halbwertszeit und somit dessen Wirksamkeit.

Rekombinantes humanes ACE2 kommt bisher als Monomer vor und ist auch als solches in der Literatur hinsichtlich seiner Funktionalität, seiner Kristallstruktur sowie hinsichtlich seiner Interaktion mit einem hochspezifischen Inhibitor beschrieben. Zur Stabilisierung des Therapeutikums wurde erfindungsgemäß ein ein Verfahren entwickelt, welches ausschließlich zu stabilen ACE2 Dimeren führt, die sowohl produktionstechnische als auch pharmakologische Vorteile im Vergleich zu ACE2 Monomeren aufweisen.

Die Dimerisierung erbringt die folgenden Vorteile:
- Bessere Löslichkeit und Bio-verfügbarkeit in Physiologischen Lösungen: Bedingt durch die stark geladenen Seitenketten, die im ACE2 Komplex nach außen gerichtet sind, ist das Dimer in ebenfalls geladenen Lösungen (zB. Physiologische Infusionslösungen, Serum, Salzlösungen,...) besser löslich als das Monomer, das ebenfalls hydrophobe Strukturen nach außen präsentieren müßte.
- Keine Aggregatbildung: Bedingt dadurch, daß sämtliche hydrophobe Proteinstrukturen in das Innere des Dimers gelangen, präsentiert der Komplex so wenig hydrophobe Abschnitte wie möglich. Dadurch kommt es zu keiner Anlagerung weiterer ACE2 Moleküle zum Dimer.
- Geringere Immunogenität: Die Immunogenität wird durch die Tatsache herabgesetzt, daß es zu keiner Aggregatbildung von ACE2 kommt. Hochmolekulare Multimere sind grundsätzlich immunogen.
- Reduzierter Proteaseangriff: Nachdem einzelne Proteinabschnitte und hierbei aller Wahrscheinlichkeit nach der C-terminale Teil in das Innere des Dimers gerichtet sind, wird dieser C-Terminus nicht abgebaut.
- Gesteigerte Halbwertszeit: Durch die geringere Immunogenität, die bessere Löslichkeit und der reduzierten Anfälligkeit für proteolytischen Abbau wird die Halbwertszeit des Proteins erhöht.
- Einfachere Proteinreinigung: Der hydrodynamische Durchmesser des ACE2 Dimers ist aufgrund seiner Struktur größer als der errechnete, nachdem sämtliche geladenen Gruppen nach außen ragen und somit zu einer ausgeprägten Solvatisierungshülle führen. Deswegen lassen sich ACE2 Dimere hervorragend von klassischen Verunreinigungen aus der Proteinexpression, wie zum Beispiel Serum Albumin (67 kDa) durch Größentrennungssäulen abtrennen.

Vorzugsweise ist das rekombinante ACE2 Polypeptid an mindestens 80% der möglichen N-Glykosylierungspositionen glykosyliert und weist einen Zuckeranteil von größer als 10% (Massen-% des gesamten ACE2) oder 11%, 12%, 13%, 14%, vorzugsweise größer als 15% oder 16%, 17%, 18%, 19%, insbesondere größer als 20 % oder 21%, 22%, 23% 24% oder 25%, auf.

Es wurde ein Produktionsprozess entwickelt, welcher hochreines und aktives vollständig komplex glykosyliertes ACE2 reproduzierbar darstellt. Das Produkt zeichnet sich aufgrund seines hohen Zuckeranteils (>20 Massen %) und der komplexen, stark verzweigten Art der teilweise negativ geladenen Zuckerstrukturen aus. Diese wirken sich positiv auf die Löslichkeit, die Bioverfügbarkeit, die Reinheit, die Aktivität sowie die Pharmakologie des Produktes aus. Durch die Wahl eines geeigneten Expressionskonstruktes, eines geeigneten Expressionswirtes, einer optimierten Selektionsstrategie, durch ein auf den Zellmetabolismus abgestimmtes Medium sowie durch minutiöse begleitende Klonanalytik und Selektion konnte eine Zelllinie hergestellt werden, die zum gewünschten Produkt führt.

ACE2 wurde bereits in Insektenzellen Sf9 und Mauszellen NSO exprimiert. Das Material wurde vor allem in in vitro Versuchen eingesetzt. Es gibt auch Resultate über transiente Expression von ACE2 in CHO. Bis jetzt wurde noch keine Zelllinie prozesstauglicher Produktivität hergestellt. Ferner wurde noch keine dementsprechende Klonselektion hinsichtlich der Produkteigenschaften, im Speziellen in Bezug auf die N-Glykosylierung, durchgeführt.

Die Löslichkeit eines Proteins wird nicht nur durch seine Aminosäuresequenz, sondern auch durch seine Faltung sowie durch post-translationelle Modifikationen bestimmt. Es sind vor allem geladene Zuckerstrukturen, die maßgeblich die Löslichkeit eines Proteins steigern und dessen pharmakologisches Profil beeinflussen. Ferner konnte für EPO aufgezeigt werden, dass die Anwesenheit von komplexen verzweigten Glykostrukturen die Halbwertszeit des Proteins positiv beinflusst.

Prinzipiell kommen zur rekombinanten Expression von ACE2 verschiedene Expressionssysteme in Frage, wobei prokaryontische Wirtszellen aufgrund fehlender Prozessierung von N-Glykosylierungsstellen nicht weiter getestet wurden. Erfolgreiche eukaryontische ACE2 Expression erfolgte bereits in Sf9 Insektenzellen und NSO Mauszellen.

Vorzugsweise weisen mindestens 70% der glykosylierten N-Glykosylierungspositionen eine Struktur unabhängig voneinander ausgewählt aus den Formeln 1-8: 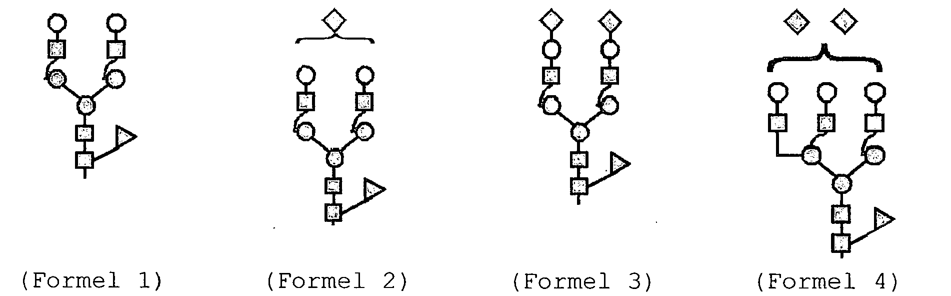 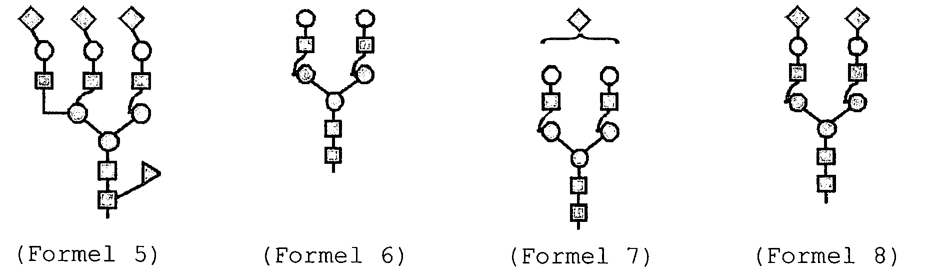 wobei
- G lc N A c: ▭
- M a n: ○
- G a l: ○

- F u c: △
- X y l: 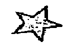
- N e u 5 A c: 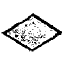
ist, auf.

Vorzugsweise sind alle der möglichen N-Glykosylierungspositionen glykosyliert.

Vorzugsweise weisen mindestens 80%, vorzugsweise mindestens 90%, insbesondere 100%, der glykosylierten N-Glykosylierungspositionen eine Struktur der Formeln 1-8 auf.

Vorzugsweise hat das Polypeptid ein Molekulargewicht von mindestens 101 kDa, vorzugsweise mindestens 105 kDa, besonders bevorzugt mindestens 109 kDa, insbesondere mindestens 113 kDa, speziell bevorzugt mindestens 117 kDa, am meisten bevorzugt mindestens 119 kDa. In anderen Ausführungsformen ist das Molekulargewicht maximal 130 kDa, 140 kDa, 150 kDa, 160 kDa, 170 kDa, 180 kDa, 190 kDa oder 200 kDa.

Vorzugsweise weist das ACE2 Polypeptid keine transmembrane Domäne auf. Es handelt sich daher um lösliches ACE2. Besonders bevorzugte Ausführungsformen umfassen dabei lösliche ACE2-Polypeptide, deren Polypeptidkette aus den Aminosäuren 18-740 oder enzymatisch aktiven Fragmenten davon. Ein weiteres Polypeptid besteht aus den Aminosäuren 18-615 der SEQ ID NO: 1.

Vorzugsweise ist ein zu Ser740 der SEQ ID NO: 1 (z.B. der C-Terminus) korrespondierendes Serin (oder C-terminale Aminosäure) des ACE2 Polypeptids O-glykosyliert.

Vorzugsweise weisen mind. 70%, vorzugsweise mind. 80%, insbesondere mind. 90%, am meisten bevorzugt 100% der glykosylierten N-Glykosylierungsstellen Sialinsäure auf, vorzugsweise sind die N-Glykosylierungsstellten entsprechend Asn53, Asn90, Asn103, Asn322, Asn432, Asn546, Asn690 der SEQ ID NO:1 sialysiert.

In speziellen Ausführungen ist ein zu Asn53 der SEQ ID NO: 1 korrespondierendes Asparagin einfach, zweifach, dreifach oder vierfach sialylisiert. In einer ACE2 Präparation sind vorzugsweise mindestens 50%, 60%, 70%, 80%, 90%, 95%, 99% oder 100% dieser Aminosäure entweder einfach, zweifach, dreifach oder vierfach sialylisiert.

In speziellen Ausführungen ist ein zu Asn90 der SEQ ID NO: 1 korrespondierendes Asparagin einfach, zweifach, dreifach oder vierfach sialylisiert. In einer ACE2 Präparation sind vorzugsweise mindestens 50%, 60%, 70%, 80%, 90%, 95%, 99% oder 100% dieser Aminosäure entweder einfach, zweifach, dreifach oder vierfach sialylisiert.

In speziellen Ausführungen ist ein zu Asn103 der SEQ ID NO: 1 korrespondierendes Asparagin einfach, zweifach, dreifach oder vierfach sialylisiert. In einer ACE2 Präparation sind vorzugsweise mindestens 50%, 60%, 70%, 80%, 90%, 95%, 99% oder 100% dieser Aminosäure entweder einfach, zweifach, dreifach oder vierfach sialylisiert.

In speziellen Ausführungen ist ein zu Asn322 der SEQ ID NO: 1 korrespondierendes Asparagin einfach, zweifach, dreifach oder vierfach sialylisiert. In einer ACE2 Präparation sind vorzugsweise mindestens 50%, 60%, 70%, 80%, 90%, 95%, 99% oder 100% dieser Aminosäure entweder einfach, zweifach, dreifach oder vierfach sialylisiert.

In speziellen Ausführungen ist ein zu Asn432 der SEQ ID NO: 1 korrespondierendes Asparagin einfach, zweifach, dreifach oder vierfach sialylisiert. In einer ACE2 Präparation sind vorzugsweise mindestens 50%, 60%, 70%, 80%, 90%, 95%, 99% oder 100% dieser Aminosäure entweder einfach, zweifach, dreifach oder vierfach sialylisiert.

In speziellen Ausführungen ist ein zu Asn546 der SEQ ID NO: 1 korrespondierendes Asparagin einfach, zweifach, dreifach oder vierfach sialylisiert. In einer ACE2 Präparation sind vorzugsweise mindestens 50%, 60%, 70%, 80%, 90%, 95%, 99% oder 100% dieser Aminosäure entweder einfach, zweifach, dreifach oder vierfach sialylisiert.

In speziellen Ausführungen ist ein zu Asn690 der SEQ ID NO: 1 korrespondierendes Asparagin einfach, zweifach, dreifach oder vierfach sialylisiert. In einer ACE2 Präparation sind vorzugsweise mindestens 50%, 60%, 70%, 80%, 90%, 95%, 99% oder 100% dieser Aminosäure entweder einfach, zweifach, dreifach oder vierfach sialylisiert.

In speziellen Ausführungen ist ein zu Ser740 der SEQ ID NO: 1 korrespondierendes Serin einfach, zweifach, dreifach oder vierfach sialylisiert. In einer ACE2 Präparation sind vorzugsweise mindestens 50%, 60%, 70%, 80%, 90%, 95%, 99% oder 100% dieser Aminosäure entweder einfach, zweifach, dreifach oder vierfach sialylisiert.

Vorzugsweise weisen mind. 30%, vorzugsweise mind. 40%, insbesondere mind. 55%, am meisten bevorzugt mind. 70% der glykosylierten N-Glykosylierungsstellen mindestens zwei Sialinsäuren auf. In einer ACE2 Präparation sind vorzugsweise mindestens 50%, 60%, 70%, 80%, 90%, 95%, 99% oder 100% dieser Aminosäure derart sialylisiert.

Vorzugsweise ist ein zu Asn53 der SEQ ID NO: 1 korrespondierendes Asparagin N-glykosyliert, vorzugsweise mit einem Glykan der Strukturen nach Formel 1, 2, 3, 4, 5, 6, 7 oder 8.

Vorzugsweise ist ein zu Asn90 der SEQ ID NO: 1 korrespondierendes Asparagin N-glykosyliert, vorzugsweise mit einem Glykan der Strukturen nach Formel 1, 2, 3, 4, 5, 6, 7 oder 8.

Vorzugsweise ist ein zu Asn103 der SEQ ID NO: 1 korrespondierendes Asparagin N-glykosyliert, vorzugsweise mit einem Glykan der Strukturen nach Formel 1, 2, 3, 4, 5, 6, 7 oder 8.

Vorzugsweise ist ein zu Asn322 der SEQ ID NO: 1 korrespondierendes Asparagin N-glykosyliert, vorzugsweise mit einem Glykan der Strukturen nach Formel 1, 2, 3, 4, 5, 6, 7 oder 8.

Vorzugsweise ist ein zu Asn432 der SEQ ID NO: 1 korrespondierendes Asparagin N-glykosyliert, vorzugsweise mit einem Glykan der Strukturen nach Formel 1, 2, 3, 4, 5, 6, 7 oder 8.

Vorzugsweise ist ein zu Asn546 der SEQ ID NO: 1 korrespondierendes Asparagin N-glykosyliert, vorzugsweise mit einem Glykan der Strukturen nach Formel 1, 2, 3, 4, 5, 6, 7 oder 8.

Vorzugsweise ist ein zu Asn690 der SEQ ID NO: 1 korrespondierendes Asparagin N-glykosyliert, vorzugsweise mit einem Glykan der Strukturen nach Formel 1, 2, 3, 4, 5, 6, 7 oder 8.

In einem weiteren Aspekt betrifft die vorliegende Erfindung eine Präparation von rekombinanten ACE2 Polypeptiden, umfassend ein Polypeptid wie hierin definiert, wobei der Anteil von ACE2 Polypeptiden mit einem Molekulargewicht von unter 100 kDa, bevorzugt von unter 104 kDa, speziell bevorzugt von unter 108 kDa, insbesondere von unter 112 kDa, besonders bevorzugt von unter 117 kDa, am meisten bevorzugt von unter 119 kDa, unter 20%, vorzugsweise unter 10%, insbesondere bevorzugt unter 5%, am meisten bevorzugt unter 1%, im Speziellen bei 0%, liegt. Der Anteil wird hierfür z.B. durch native Gelelektrophorese bestimmt.

Vorzugsweise liegt der Anteil von ACE2 Polypeptiden mit Transmembrandomänen unter 20%, vorzugsweise unter 10%, insbesondere bevorzugt unter 5%, am meisten bevorzugt unter 1%, im Speziellen bei 0%.

Vorzugsweise liegt der Anteil von ACE2 Multimeren unter 20%, vorzugsweise unter 10%, insbesondere bevorzugt unter 5%, am meisten bevorzugt unter 1%, im Speziellen bei 0%. Unter ACE2 Multimeren werden Komplexe mit 3 oder mehr ACE2 Polypeptiden verstanden.

Vorzugsweise beträgt der Anteil an ACE2 Dimeren an ACE2 Molekülen mindestens 10%, 20%, 30%, 40% 50%, 60%, 70%, 80%, 90%, 95% oder mindestens 99%, sein. In weiteren Ausführungsformen kann in Kombination dazu oder unabhängig der Anteil an ACE2 Monomeren an ACE2 Molekülen mindestens 10%, 20%, 30%, 40% 50%, 60%, 70%, 80%, 90%, 95% oder mindestens 99%, sein.

Vorzugsweise ist der Anteil an ACE2 Polypeptiden mit N-glykosyliertem Asparagin entsprechend Asn53 der SEQ ID NO: 1 größer als 60%, vorzugsweise größer als 70%, insbesondere bevorzugt größer als 80%, speziell bevorzugt größer als 90%, am meisten bevorzugt größer als 99%, insbesondere 100%, und vorzugsweise mit einem Glykan der Strukturen nach Formel 1, 2, 3, 4, 5, 6, 7 oder 8.

Vorzugsweise ist der Anteil an ACE2 Polypeptiden mit N-glykosyliertem Asparagin entsprechend Asn90 der SEQ ID NO: 1 größer als 60%, vorzugsweise größer als 70%, insbesondere bevorzugt größer als 80%, speziell bevorzugt größer als 90%, am meisten bevorzugt größer als 99%, insbesondere 100%, und vorzugsweise mit einem Glykan der Strukturen nach Formel 1, 2, 3, 4, 5, 6, 7 oder 8.

Vorzugsweise ist der Anteil an ACE2 Polypeptiden mit N-glykosyliertem Asparagin entsprechend Asn103 der SEQ ID NO: 1 größer als 60%, vorzugsweise größer als 70%, insbesondere bevorzugt größer als 80%, speziell bevorzugt größer als 90%, am meisten bevorzugt größer als 99%, insbesondere 100%, und vorzugsweise mit einem Glykan der Strukturen nach Formel 1, 2, 3, 4, 5, 6, 7 oder 8.

Vorzugsweise ist der Anteil an ACE2 Polypeptiden mit N-glykosyliertem Asparagin entsprechend Asn322 der SEQ ID NO: 1 größer als 60%, vorzugsweise größer als 70%, insbesondere bevorzugt größer als 80%, speziell bevorzugt größer als 90%, am meisten bevorzugt größer als 99%, insbesondere 100%, und vorzugsweise mit einem Glykan der Strukturen nach Formel 1, 2, 3, 4, 5, 6, 7 oder 8.

Vorzugsweise ist der Anteil an ACE2 Polypeptiden mit N-glykosyliertem Asparagin entsprechend Asn432 der SEQ ID NO: 1 größer als 60%, vorzugsweise größer als 70%, insbesondere bevorzugt größer als 80%, speziell bevorzugt größer als 90%, am meisten bevorzugt größer als 99%, insbesondere 100%, und vorzugsweise mit einem Glykan der Strukturen nach Formel 1, 2, 3, 4, 5, 6, 7 oder 8.

Vorzugsweise ist der Anteil an ACE2 Polypeptiden mit N-glykosyliertem Asparagin entsprechend Asn546 der SEQ ID NO: 1 größer als 60%, vorzugsweise größer als 70%, insbesondere bevorzugt größer als 80%, speziell bevorzugt größer als 90%, am meisten bevorzugt größer als 99%, insbesondere 100%, und vorzugsweise mit einem Glykan der Strukturen nach Formel 1, 2, 3, 4, 5, 6, 7 oder 8.

Vorzugsweise ist der Anteil an ACE2 Polypeptiden mit N-glykosyliertem Asparagin entsprechend Asn690 der SEQ ID NO: 1 größer als 60%, vorzugsweise größer als 70%, insbesondere bevorzugt größer als 80%, speziell bevorzugt größer als 90%, am meisten bevorzugt größer als 99%, insbesondere 100%, und vorzugsweise mit einem Glykan der Strukturen nach Formel 1, 2, 3, 4, 5, 6, 7 oder 8.

Vorzugsweise ist der Anteil an ACE2 Polypeptiden mit O-glykosyliertem Serin entsprechend Ser740 der SEQ ID NO: 1 größer als 60%, vorzugsweise größer als 70%, insbesondere bevorzugt größer als 80%, speziell bevorzugt größer als 90%, am meisten bevorzugt größer als 99%, insbesondere 100%.

Vorzugsweise ist die katalytische Aktivität des ACE2 Polypeptids oder der Präparation kkat mindestens 4 /s, vorzugsweise mindestens 5 /s, insbesondere bevorzugt mindesten 6 /s, speziell bevorzugt mindestens 7 /s, am meisten bevorzugt mindestens 7,6 /s, bezogen auf den Ang 1-7 (Angiotensin 1-7) Umsatz. Der Umsatz kann wie in den Beispielen beschrieben auf simple Weise getestet werden.

In einem weiteren Aspekt betrifft die vorliegende Erfindung ein Verfahren zur Herstellung von rekombinanten ACE2 Polypeptiden oder einer Präparation von rekombinanten ACE2, umfassend die Schritte der Einbringung eines ACE2 codierenden Polynukleotides, vorzugsweise eines ACE2 ohne Transmembrandomäne codierenden Polynukleotids, in eukaryotischen Zellen, Expression des ACE2 Polypeptids und Sammeln des exprimierten ACE2, insbesondere in dimerer Form . Die Zellen können danach selektiert werden, ein erfindungsgemäßes ACE2 Polypeptid, wie hierin beschrieben, insbesondere mit einem hohen Molekulargewicht, zu produzieren.

Vorzugsweise ist das ACE2 codierende Polynukleotid auf einem Vektor vorgesehen.

Vorzugsweise wird die ACE2-Expression mit einem Marker, vorzugsweise DHFR, selektiert. Vorzugsweise liegt der Marker auf dem Vektor vor.

Vorzugsweise weist der Vektor eine IRES für die exprimierte ACE2 mRNA auf (oder codiert hierfür).

Vorzugsweise sind die eukaryotischen Zellen CHO Zellen.

Die vorliegende Erfindung betrifft auch rekombinantes ACE2 Polypeptid oder eine Präparation von rekombinanten ACE2 Polypeptiden erhältlich durch ein solches Verfahren.

In einem weiteren Aspekt liefert die Erfindung eine stabil mit einem ACE2 codierenden Polynukleotid transfizierte eukaryontische Zelllinie (oder Zelle), vorzugsweise CHO Zelllinie (oder Zelle), die ACE2, insbesondere wie oben definiert, exprimiert.

Die Zelle weist vorzugsweise eine ACE2-Produktivität von mindestens 10 pg/Zelle/Tag, vorzugsweise mindestens 15 pg/Zelle/Tag, insbesondere bevorzugt von mindestens 20 pg/Zelle/Tag, auf.

Besonders bevorzugt sind folgende Definitionen der Erfindung:
1. Rekombinantes ACE2 Polypeptid, dadurch gekennzeichnet, dass es glykosiliert ist, wobei die Glycogruppen eines ACE2 Polypeptid Monomers in Summe mindestens 14 Sialylsäure-Reste aufweisen und das ACE2 Polypetid als Dimer vorliegt.
2. Rekombinantes ACE2 Polypeptid nach Definition 1, dadurch gekennzeichnet, dass es an mindestens 80% der möglichen N-Glykosylierungspositionen glykosyliert ist und einen Zuckeranteil von größer als 10% (Massen-%, bezogen auf das gesamte ACE2-Molekül), vorzugsweise größer als 15%, insbesondere größer als 20 %, aufweist.
3. Rekombinantes ACE2 Polypeptid nach Definition 1 oder 2, dadurch gekennzeichnet, dass mindestens 70% der glykosylierten N-Glykosylierungspositionen eine Struktur unabhängig voneinander ausgewählt aus den Formeln 1-8: 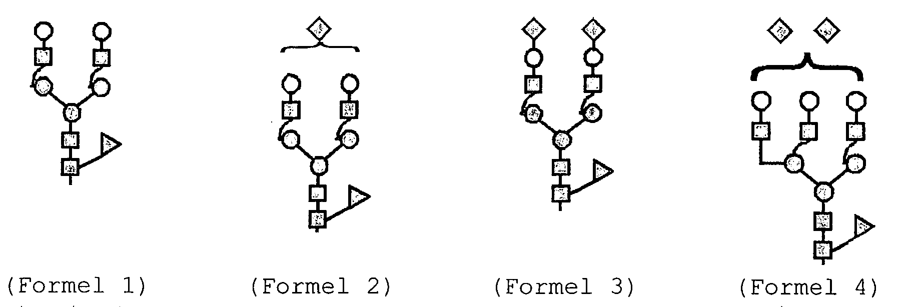 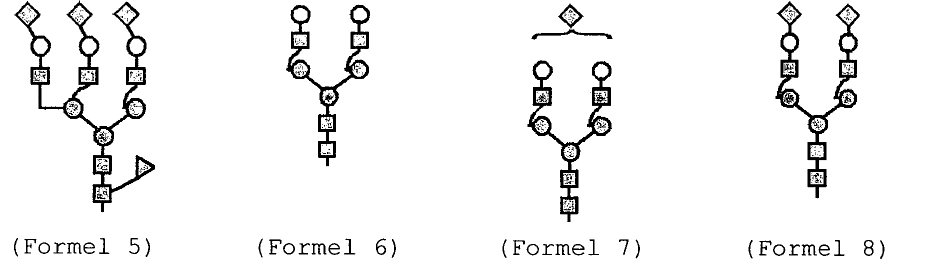 wobei
   - G lc N A c: ▭
   - M a n: ○
   - G a l: ○
   - F u c: △
   - X y l: 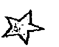
   - N e u 5 A c: 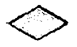
   ist, aufweisen.
4. Rekombinantes ACE2 Polypeptid nach einer der Defintionen 1 bis 3, dadurch gekennzeichnet, dass alle der möglichen N-Glykosylierungspositionen glykosyliert sind.
5. Rekombinantes ACE2 Polypeptid nach einer der Defintionen 1 bis 4, dadurch gekennzeichnet, dass mindestens 80%, vorzugsweise mindestens 90%, insbesondere 100%, der glykosylierten N-Glykosylierungspositionen eine Struktur der Formeln 1-8 aufweisen.
6. Rekombinantes ACE2 Polypeptid nach einer der Definitionen 1 bis 5, dadurch gekennzeichnet, dass das ACE2 Monomer ein Molekulargewicht von mindestens 101 kDa, vorzugsweise mindestens 105 kDa, besonders bevorzugt mindestens 109 kDa, insbesondere mindestens 113 kDa, speziell bevorzugt mindestens 117 kDa, am meisten bevorzugt mindestens 119 kDa, aufweist.
7. Rekombinantes ACE2 Polypeptid nach einer der Definitionen 1 bis 6, dadurch gekennzeichnet, dass das ACE2 Polypeptid keine transmembrane Domäne aufweist, vorzugsweise dass die ACE2 Polypeptidkette aus den Aminosäuren 18-740 von SEQ ID NO: 1 oder enzymatisch aktiven Fragmenten davon besteht.
8. Rekombinantes ACE2 Polypeptid nach einer der Definitionen 1 bis 7, dadurch gekennzeichnet, dass ein zu Ser740 der SEQ ID NO: 1 korrespondierendes Serin O-glykosyliert ist.
9. Rekombinantes ACE2 Polypeptid nach einer der Definitionen 1 bis 8, dadurch gekennzeichnet, dass mind. 70%, vorzugsweise mind. 80%, insbesondere mind. 90%, am meisten bevorzugt 100% der glykosylierten N-Glykosylierungsstellen Sialinsäure aufweisen, vorzugsweise sind die N-Glykosylierungsstellen entsprechend Asn53, Asn90, Asn103, Asn322, Asn432, Asn546, Asn690 der SEQ ID NO: 1 sialysiert.
10. Rekombinantes ACE2 Polypeptid nach einer der Definitionen 1 bis 9, dadurch gekennzeichnet, dass mind. 30%, vorzugsweise mind. 40%, insbesondere mind. 55%, am meisten bevorzugt mind. 70% der glykosylierten N-Glykosylierungsstellen mindestens zwei Sialinsäuren aufweisen.
11. Rekombinantes ACE2 Polypeptid nach einer der Definitionen 1 bis 9, dadurch gekennzeichnet, dass ein zu Asn53 der SEQ ID NO: 1 korrespondierendes Asparagin N-glykosyliert ist, vorzugsweise mit einem Glykan der Strukturen nach Formel 1, 2, 3, 4, 5, 6, 7 oder 8 wie in Definition 3 definiert.
12. Rekombinantes ACE2 Polypeptid nach einer der Definitionen 1 bis 11, dadurch gekennzeichnet, dass ein zu Asn90 der SEQ ID NO: 1 korrespondierendes Asparagin N-glykosyliert ist, vorzugsweise mit einem Glykan der Strukturen nach Formel 1, 2, 3, 4, 5, 6, 7 oder 8 wie in Definition 3 definiert.
13. Rekombinantes ACE2 Polypeptid nach einer der Definitionen 1 bis 12, dadurch gekennzeichnet, dass ein zu Asn103 der SEQ ID NO: 1 korrespondierendes Asparagin N-glykosyliert ist, vorzugsweise mit einem Glykan der Strukturen nach Formel 1, 2, 3, 4, 5, 6, 7 oder 8 wie in Definition 3 definiert.
14. Rekombinantes ACE2 Polypeptid nach einer der Definitionen 1 bis 13, dadurch gekennzeichnet, dass ein zu Asn322 der SEQ ID NO: 1 korrespondierendes Asparagin N-glykosyliert ist, vorzugsweise mit einem Glykan der Strukturen nach Formel 1, 2, 3, 4, 5, 6, 7 oder 8 wie in Definition 3 definiert.
15. Rekombinantes ACE2 Polypeptid nach einer der Definitionen 1 bis 14, dadurch gekennzeichnet, dass ein zu Asn432 der SEQ ID NO: 1 korrespondierendes Asparagin N-glykosyliert ist, vorzugsweise mit einem Glykan der Strukturen nach Formel 1, 2, 3, 4, 5, 6, 7 oder 8 wie in Definition 3 definiert.
16. Rekombinantes ACE2 Polypeptid nach einer der Definitionen 1 bis 15, dadurch gekennzeichnet, dass ein zu Asn546 der SEQ ID NO: 1 korrespondierendes Asparagin N-glykosyliert ist, vorzugsweise mit einem Glykan der Strukturen nach Formel 1, 2, 3, 4, 5, 6, 7 oder 8 wie in Definition 3 definiert.
17. Rekombinantes ACE2 Polypeptid nach einer der Definitionen 1 bis 16, dadurch gekennzeichnet, dass ein zu Asn690 der SEQ ID NO: 1 korrespondierendes Asparagin N-glykosyliert ist, vorzugsweise mit einem Glykan der Strukturen nach Formel 1, 2, 3, 4, 5, 6, 7 oder 8 wie in Definition 3 definiert.
18. Präparation von rekombinanten ACE2 Polypeptiden, umfassend ein Polypeptid nach einer der Definitionen 1 bis 17, dadurch gekennzeichnet, dass der Anteil von ACE2 Polypeptiden mit einem Molekulargewicht von unter 100 kDa, bevorzugt von unter 104 kDa, speziell bevorzugt von unter 108 kDa, insbesondere von unter 112 kDa, besonders bevorzugt von unter 117 kDa, am meisten bevorzugt von unter 119 kDa, unter 20%, vorzugsweise unter 10%, insbesondere bevorzugt unter 5%, am meisten bevorzugt unter 1%, im speziellen bei 0%, liegt.
19. Präparation nach Definition 18, dadurch gekennzeichnet, dass der Anteil von ACE2 Polypeptiden mit Transmembrandomänen unter 20%, vorzugsweise unter 10%, insbesondere bevorzugt unter 5%, am meisten bevorzugt unter 1%, im Speziellen bei 0%, liegt.
20. Präparation nach Definition 18 oder 19, dadurch gekennzeichnet dass der Anteil von ACE2 Multimeren unter 20%, vorzugsweise unter 10%, insbesondere bevorzugt unter 5%, am meisten bevorzugt unter 1%, im speziellen bei 0%, liegt.
21. Präparation nach einer der Definitionen 18 bis 20, dadurch gekennzeichnet, dass der Anteil an ACE2 Dimeren an ACE2 Molekülen mindestens 70%, vorzugsweise mindestens 80%, besonders bevorzugt mindestens 90% im speziellen bevorzugt mindestens 95% am meisten bevorzugt mindestens 99%, ist.
22. Präparation nach einer der Definitionen 18 bis 20, dadurch gekennzeichnet, dass der Anteil an ACE2 Polypeptiden mit N-glykosyliertem Asparagin entsprechend Asn53 der SEQ ID NO: 1 größer als 60%, vorzugsweise größer als 70%, insbesondere bevorzugt größer als 80%, speziell bevorzugt größer als 90%, am meisten bevorzugt größer als 99%, insbesondere 100%, ist und vorzugsweise mit einem Glykan der Strukturen nach Formel 1, 2, 3, 4, 5, 6, 7 oder 8 wie in Definition 3 definiert.
23. Präparation nach einer der Definitionen 18 bis 22, dadurch gekennzeichnet, dass der Anteil an ACE2 Polypeptiden mit N-glykosyliertem Asparagin entsprechend Asn90 der SEQ ID NO: 1 größer als 60%, vorzugsweise größer als 70%, insbesondere bevorzugt größer als 80%, speziell bevorzugt größer als 90%, am meisten bevorzugt größer als 99%, insbesondere 100%, ist und vorzugsweise mit einem Glykan der Strukturen nach Formel 1, 2, 3, 4, 5, 6, 7 oder 8 wie in Definition 3 definiert.
24. Präparation nach einer der Definitionen 18 bis 23, dadurch gekennzeichnet, dass der Anteil an ACE2 Polypeptiden mit N-glykosyliertem Asparagin entsprechend Asn103 der SEQ ID NO: 1 größer als 60%, vorzugsweise größer als 70%, insbesondere bevorzugt größer als 80%, speziell bevorzugt größer als 90%, am meisten bevorzugt größer als 99%, insbesondere 100%, ist und vorzugsweise mit einem Glykan der Strukturen nach Formel 1, 2, 3, 4, 5, 6, 7 oder 8 wie in Definition 3 definiert.
25. Präparation nach einer der Definitionen 18 bis 24, dadurch gekennzeichnet, dass der Anteil an ACE2 Polypeptiden mit N-glykosyliertem Asparagin entsprechend Asn322 der SEQ ID NO: 1 größer als 60%, vorzugsweise größer als 70%, insbesondere bevorzugt größer als 80%, speziell bevorzugt größer als 90%, am meisten bevorzugt größer als 99%, insbesondere 100%, ist und vorzugsweise mit einem Glykan der Strukturen nach Formel 1, 2, 3, 4, 5, 6, 7 oder 8 wie in Definition 3 definiert.
26. Präparation nach einer der Definitionen 18 bis 25, dadurch gekennzeichnet, dass der Anteil an ACE2 Polypeptiden mit N-glykosyliertem Asparagin entsprechend Asn432 der SEQ ID NO: 1 größer als 60%, vorzugsweise größer als 70%, insbesondere bevorzugt größer als 80%, speziell bevorzugt größer als 90%, am meisten bevorzugt größer als 99%, insbesondere 100%, ist und vorzugsweise mit einem Glykan der Strukturen nach Formel 1, 2, 3, 4, 5, 6, 7 oder 8 wie in Definition 3 definiert.
27. Präparation nach einer der Definitionen 18 bis 26, dadurch gekennzeichnet, dass der Anteil an ACE2 Polypeptiden mit N-glykosyliertem Asparagin entsprechend Asn546 der SEQ ID NO: 1 größer als 60%, vorzugsweise größer als 70%, insbesondere bevorzugt größer als 80%, speziell bevorzugt größer als 90%, am meisten bevorzugt größer als 99%, insbesondere 100%, ist und vorzugsweise mit einem Glykan der Strukturen nach Formel 1, 2, 3, 4, 5, 6, 7 oder 8 wie in Definition 3 definiert.
28. Präparation nach einer der Definitionen 18 bis 27, dadurch gekennzeichnet, dass der Anteil an ACE2 Polypeptiden mit N-glykosyliertem Asparagin entsprechend Asn690 der SEQ ID NO: 1 größer als 60%, vorzugsweise größer als 70%, insbesondere bevorzugt größer als 80%, speziell bevorzugt größer als 90%, am meisten bevorzugt größer als 99%, insbesondere 100%, ist und vorzugsweise mit einem Glykan der Strukturen nach Formel 1, 2, 3, 4, 5, 6, 7 oder 8 wie in Definition 3 definiert.
29. Präparation nach einer der Definitionen 18 bis 28, dadurch gekennzeichnet, dass der Anteil an ACE2 Polypeptiden mit O-glykosyliertem Serin entsprechend Ser740 der SEQ ID NO: 1 größer als 60%, vorzugsweise größer als 70%, insbesondere bevorzugt größer als 80%, speziell bevorzugt größer als 90%, am meisten bevorzugt größer als 99%, insbesondere 100%, ist.
30. ACE2 Polypeptid oder Präparation nach einer der Definitionen 1 bis 29 mit einer katalytischen Aktivität kkat von mindestens 4 /s, vorzugsweise von mindestens 5 /s, insbesondere bevorzugt von mindestens 6 /s, speziell bevorzugt von mindestens 7 /s, am meisten bevorzugt von mindestens 7,6 /s, bezogen auf den Ang 1-7 (Angiotensin 1-7) Umsatz.
31. Verfahren zur Herstellung von rekombinanten ACE2 Polypeptiden oder einer Präparation von rekombinantem ACE2, vorzugsweise nach einer der Definitionen 1 bis 30, umfassend die Schritte der Einbringung eines ACE2 codierenden Polynukleotides, vorzugsweise eines ACE2 ohne Transmembrandomäne codierenden Polynukleotides, in eukaryontischen Zellen, Expression des ACE2 Polypeptids und Sammeln des exprimierten ACE2, insbesondere in dimerer Form, wobei vorzugsweise bei der Expression die Zn²⁺-Konzentration mindestens 1,5 micromolar ist. Die Zn²⁺-Konzentration z.B. im Nahrmedium der exprimierten Zellen kann mindestens 0,5, 0,75, 1,0, 1,25, 1,5, 1,75, 2,0, 2,25 oder mindestens 2,5 oder 3,0 sein.
32. Verfahren nach Definition 31, dadurch gekennzeichnet, dass das ACE2 codierende Polynukleotid auf einem Vektor vorgesehen ist.
33. Verfahren nach Definition 31 oder 32, dadurch gekennzeichnet, dass die ACE2-Expression mit einem Marker, vorzugsweise DHFR, selektiert wird.
34. Verfahren nach Definition 33, dadurch gekennzeichnet, dass der Marker auf dem Vektor vorliegt.
35. Verfahren nach Definition 34, dadurch gekennzeichnet, dass der Vektor eine IRES für die exprimierte ACE2 mRNA aufweist.
36. Verfahren nach einer der Definitionen 31 bis 35, dadurch gekennzeichnet, dass die eukaryontischen Zellen CHO Zellen sind.
37. Rekombinantes ACE2 Polypeptid oder Präparation von rekombinanten ACE2 Polypeptiden erhältlich durch ein Verfahren der vorhergehenden Definitionen.
38. Stabil mit einem ACE2 codierenden Polynukleotid transfizierte eukaryontische Zellinie, vorzugsweise CHO Zelllinie, die ACE2 nach einer der Definitionen 1 bis 30 exprimiert.
39. Zelle nach Definition 38, die eine ACE2-Produktivität von mindestens 10 pg/Zelle/Tag, vorzugsweise mindestens 15 pg/Zelle/Tag, insbesondere bevorzugt von mindestens 20 pg/Zelle/Tag aufweist.

Die vorliegende Erfindung wird durch die folgenden Figuren und Beispiele weiter illustriert ohne auf diese beschränkt zu sein. Figuren:
Fig.1: ACE2 Expressions- und Selektionskassette
Fig.2: ACE2 spezifische Western Blot Analyse der Produktionsklongeschichte
Fig.3: SDS-PAGE Analytik des ACE2 Monomers
Fig.4: Klonauswahl

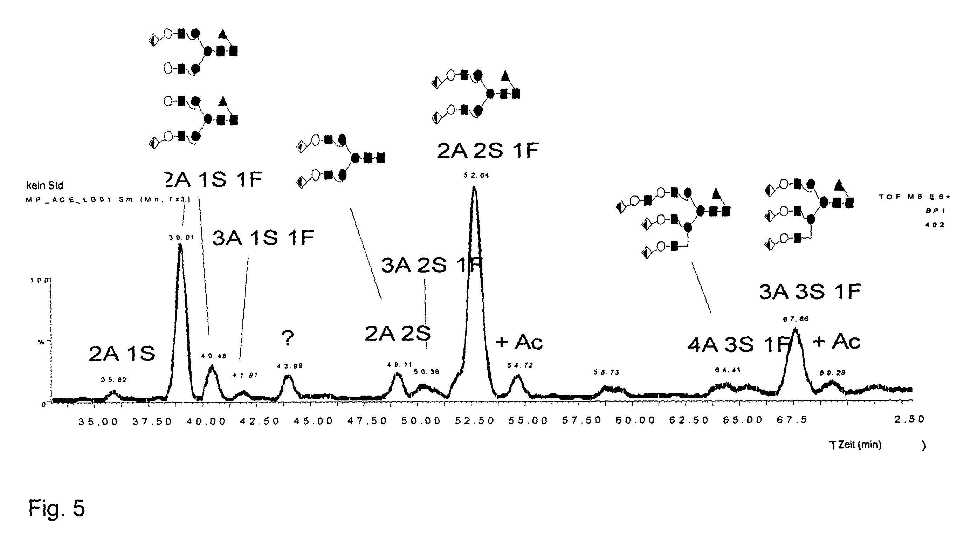

Fig.5: LC-MS Glykosylierungsanalytik
Fig.6: Präparative Größentrennung von ACE2
Fig.7: Pharmakokinetik von ACE2 in 3 Spezies
Fig.8: Kalibrationskurven zur Ang 1-7 und Ang II Quantifizierung. Die Peptide wurden im angeführten Konzentrationsbereich mittels RP-HPLC auf Waters C18 µBondapak RP, 2,1x300mm, 10µm, 125Å Säule aufgetrennt.
Fig. 9: MS/MS-Spektrum des N-terminalen Peptids. Anmerkung: Q und K haben die gleiche Masse.
Fig. 10: Sequenz von ACE2 und N-Glykosylierungs-Vorhersage
Fig. 11: aufgeschlüsseltes Spektrum für Glykosylierungsstellen 103 (A), Stelle 432 (B), Stelle 546 (C), Stelle 690 (D), Stelle 90 (E).
Fig. 12: Spektrum des C-terminalen O-glykosylierten Peptids. Die strukturelle Zuordnung muss als probeweise klassifiziert werden.
Fig. 13: LC-MS freigesetzter Glykane.
Fig. 14: Bestimmung der ACE2 Dimerstruktur mittels nativem PAGE (links, Proteinbanden wurden mittels Silberfärbung visualisiert) und SEC (rechts, Trennung auf einer Zorbax G-450 Säule in Gegenwart von 220 mM Na-Phosphat bei pH 7.0 in 10% Acetonitril, das Chromatogram wurde bei 214 nm aufgenommen.
Fig. 15: Chromatogramm einer Größenausschlusschromatographie des Dimeren ACE2 (Retention 8,55 min, 8,93 min). Standard: Thyroglobulin (670 kDa, 7,43 min), Gamma-globulin (158 kDa), Ovalbumin (43 kDa, 10,08 min), Myoglobulin (17 kDa, 11,08 min), Vitamin B-12 (1,3 kDa, 12,71 min).

### Beispiele:

### Beispiel 1: Expression von hochglykosyliertem ACE2

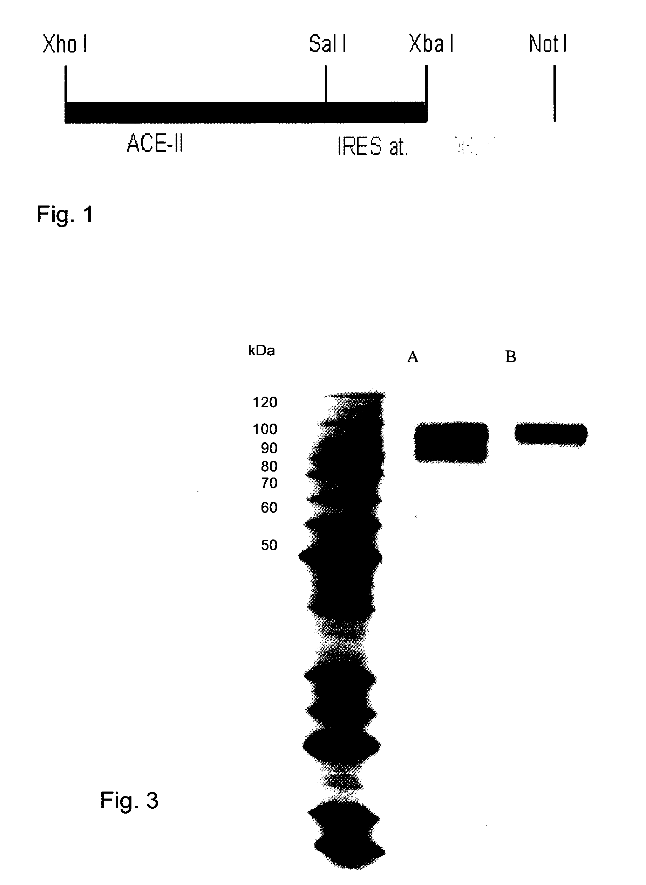

Der lösliche Abschnitt der humanen ACE2 Sequenz (SEQ ID NO: 1) wurde in einen Expressionsvektor kloniert, in welchen zuvor der amplifizierbare Selektionsmarker DHFR eingefügt wurde, um zu einer erhöhten Expression des ACE2 Genes zu führen. Hierzu wurde zwischen die für ACE2 und DFHR kodierenden Gene eine attenuierte IRES eingebracht, welche die bi-cistronische Ablesung von ACE2 und DHFR auf derselben mRNA ermöglicht. Die ACE2 Expressions- und Selektionskassette ist in Figur 1 graphisch dargestellt. Nachdem beide Proteine unter Kontrolle desselben Promotors exprimiert werden, kann über DHFR Selektion unter Verwendung des Antagonisten MTX zielstrebig die ACE2 Expression gesteigert werden. Durch diese Strategie ist es möglich, besonders stabile Expressionszelllinien zu erhalten, die hohe Ausbeuten eines Produktes konstanter Qualität liefern. Dies ermöglicht es auch in Zelllinien vernünftige Produkttiter zu erzielen, die möglicherweise für die rekombinante Expression eines bestimmten Targetproteins minder geeignet sind.

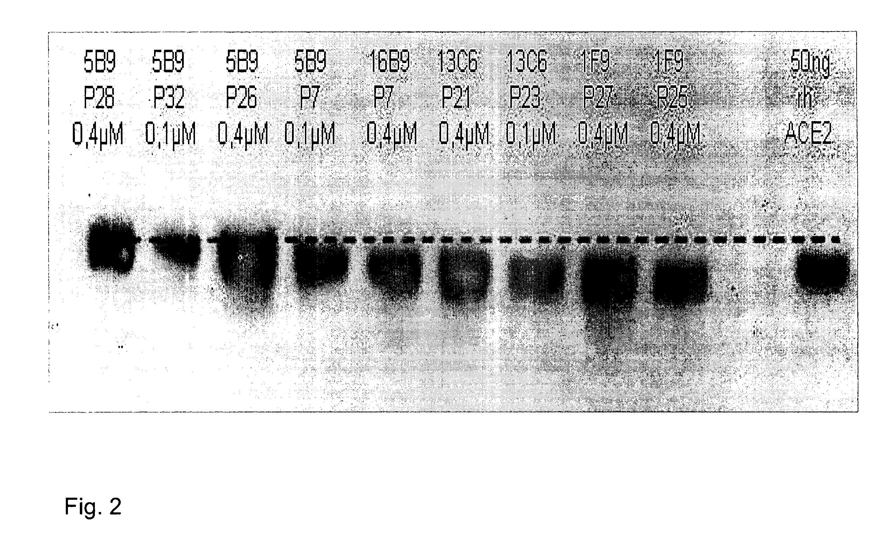

Dieser Vektor wurde in CHOdhfr- transfiziert und unter kontinuierlich gesteigertem MTX Druck wurde die Kopienanzahl der ACE2 Gene amplifiziert. Über mehrere Selektions- und Subklonierungsrunden wurden die besten Produzenten mittels intrazellulärer FACS Analytik und proteinchemischer sowie enzymatischer Analysen auf optimale Produkteigenschaften selektiert: Es wurden für die Auswahl des bestgeeigneten Klons vor allem die spezifische enzymatische Aktivität, welche mit 3 verschiedenen Substraten gemessen wurde, die Produkthomogenität, die zelluläre Produktivität jedoch auch die Zuckerkomplexizität in Betracht gezogen. In Fig. 2 ist eine rekapitulative Western Blot Analyse der sukzessiven Kulturüberstände einzelner Klone dargestellt, die zur Etablierung der Produktionszelllinie verwendet wurden. Die Produkteigenschaften der einzelnen Klone unterscheiden sich insofern, dass der Zuckeranteil der Expressionsprodukte von rechts nach links zunimmt, was durch eine deutliche Massenzunahme erkennbar ist. Dies wurde durch spezifische Selektion hochglykosylierender Klone erreicht. (Letztendlich wurde Klon 5B9, welcher das Expressionsprodukt mit dem höchstem Molekulargewicht aufwies, zur Etablierung der Produktionszelllinie 1B4 verwendet.)

Es wurde entschieden, 6 Klone weiterzuführen, die enzymatisch hoch aktives und komplex N-glykosyliertes ACE2 exprimierten. Während lösliches ACE2 ein Molekulargewicht von 83 kDa aufweist, werden Klone ausgewählt, welche im SDS-PAGE, bedingt durch deren Zuckerstruktur, im Bereich von bis zu 120 kDa erschienen. Fig. 3 zeigt ein durch den vorliegenden Produktionsprozess hergestelltes ACE2 (Lane B) im Vergleich zu einem in NSO hergestellten ACE2 Standard (Lane A). Während das erfindungsgemäße ACE2 Polypeptid - hier als Monomer analysiert - homogen, hoch glykosyliert ist und daher als einzelne Bande bei ca. 120 kDa erscheint weist das Referenzmaterial Banden von 83 kDa bis zu 120 kDa auf, was eine sehr heterogene Glykosylierung andeutet.

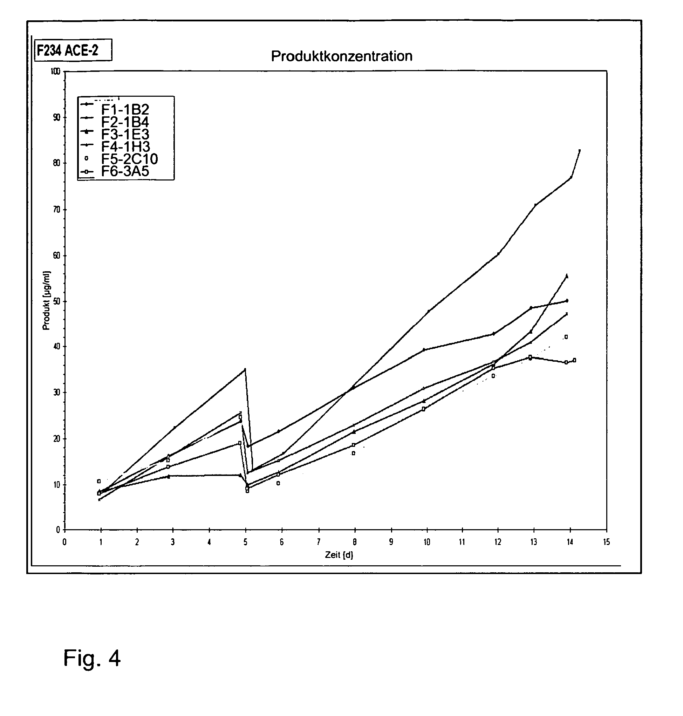

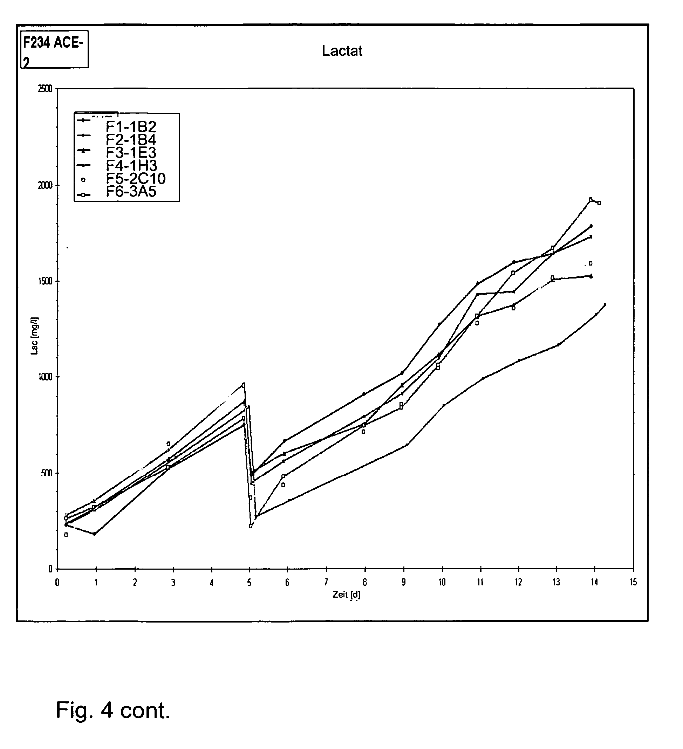

Die präliminären Klone wurden im Weiteren auf proteinfreies Wachstumsmedium umgestellt. Dieses Medium ist chemisch definiert und auf die rekombinante Expression von Glykoproteinen in CHO abgestimmt worden. Alle 6 Klone wurden in Kultur gehalten und auf deren Produktionsprozesstauglichkeit überprüft. Im Speziellen wurden Wachstumsraten aufgezeichnet sowie die Überstände auf Produktverlauf und Metaboliten untersucht (Fig. 4). Wiederum wurden die Expressionsprodukte sowie die Klone genau analysiert. Alle exprimierten hochaktives ACE2 und wiesen Produktivitäten um 20-30 pg/Zelle/Tag auf. Ferner wurden ebenfalls die Zuckerstrukturen und deren Heterogenität analysiert. Letztendlich wurde Klon 1B4 ausgewählt. Er wies über den gesamten Produktionsverlauf eine homogene Zuckerstruktur auf. Es waren alle 7 N-Glykosylierungsstellen prozessiert, wobei diese mindestens eine bi-, manche jedoch auch eine tri-antennäre komplexe Glykosylierung mit terminalen Sialinsäuren aufwiesen.

Auf der Basis dieses Klones wurde eine Mastercellbank hergestellt und getestet, sowie ein GMP tauglicher Reinigungs- und in weiterer Folge ein GMP Produktionsprozess aufgebaut.

SEQ ID NO: 1 (ACE2 Proteinsequenz, die autologe Signalsequenz (unterstrichen) wird durch die Wirtszelle für die Ausschleusung abgespaltet):

MSSSSWLLLSLVAVTAA
QSTIEEQAKTFLDKFNHEAEDLFYQSSLASWNYNTNITEENVQNMNNAGDKWSAFLKEQST-
LAQMYPLQEIQNLTVKLQLQALQQNGSSVLSEDKSKRLNTILNTMSTIYSTGKVCNPDNPQE-
CLLLEPGLNEIMANSLDYNERLWAWESWRSEVGKQLRPLYEEYVVLKNEMARANHYEDYGDYWR
GDYEVNGVDGYDYSRGQLIEDVEHTFEEIKPLYEHLHAYVRAKLMNAYPSYISPIGCL-
PAHLLGDMWGRFWTNLYSLTVPFGQKPNIDVTDAMVDQAWDAQRIFKEAEKFFVSVGLPNMT-
QGFWENSMLTDPGNVQKAVCHPTAWDLGKGDFRILMCTKVTMDDFLTAHHEMGHIQYDMAYAAQ
PFLLRNGANEGFHEAVGEIMSLSAATPKHLKSIGLLSPDFQEDNETEINFLLKQAL-
TIVGTLPFTYMLEKWRWMVFKGEIPKDQWMKKWWEMKREIVGVVEPVPHDETYCDPASLF-
HVSNDYSFIRYYTRTLYQFQFQEALCQAAKHEGPLHKCDISNSTEAGQKLFNMLRLGKSEPWTL
ALENVVGAKNMNVRPLLNYFEPLFTWLKDQNKNSFVGWSTDWSPYADQSIKVRISLKSALGD-
KAYEWNDNEMYLFRSSVAYAMRQYFLKVKNQMILFGEEDVRVANLKPRISFNFFVTAPKNVS-
DIIPRTEVEKAIRMSRSRINDAFRLNDNSLEFLGIQPTLGPPNQPPVS

### Beispiel 2: Produkteigenschaften

Komplexes, hochglykosylieres rhACE2 weist aufgrund der kovalent gebundenen Zuckerstrukturen ein deutlich höheres Molekulargewicht auf. So wurde mittels Peptidemap eine Molmasse von 102 kDa gemessen, während die von nicht-glykosyliertem ACE2 nur 83 kDa beträgt. Der Zuckeranteil liegt dementsprechend bei 23% und ist für die im Weiteren beschriebenen Produkteigenschaften von essentieller Bedeutung.

Nicht das natürliche ACE2 wurde exprimiert, sondern ausschließlich der lösliche Anteil von ACE2. Aufgrund der fehlenden Membrandomäne wird es vollständiger glykosyliert. Ferner wurde eine zellinterne Signalweiterleitung nach extrazellulärer Stimulation von ACE2 ausgeschlossen, welche möglicherweise über den cytoplasmischen Anteil des membrangebundenen, natürlichen ACE2 erfolgt.

Aufgrund der komplexen, hoch-sialylierten und somit stark negativ geladenen Zuckerstrukturen besitzt ACE2 eine deutlich höhere Löslichkeit im Vergleich zu nicht glykosyliertem oder unvollständig glykosyliertem ACE2. Somit können physiologisch gepufferte Proteinformulierungen von bis zu 15 mg/ml problemlos hergestellt werden.

Das Produkt verhält sich ferner auch stabil in Lösung und verliert über einen längeren Lagerungszeitraum weder Aktivität, noch neigt es zum Abbau oder zur Aggregation, abgesehen von der Dimerisierung. Deglykosyliertes ACE2 hingegen fällt bereits im geringen Konzentrationsbreich aus. Dies konnte in einem präparativen Deglykosylierungsansatz mittels PNGaseF gezeigt werden. Der für ACE2 berechnete, theoretische Stabilitätsindex beträgt 41,2 und klassifiziert das Protein als instabil, wobei die Zuckerstrukturen außer Acht gelassen wurden. Nachdem sich diese Formulierung jedoch monatelang stabil in Lösung verhält, ist dies auf den hohen Zuckeranteil zurückzuführen. Die bessere Solvatisierung von ACE2 führt ferner dazu, dass diese Formulierung ausschließlich aus ACE2 Dimeren (bzw. nach Trennung zum Monomer ausschließlich aus Monomeren) besteht, während nicht glykosyliertes ACE2 zur Multimerisierung und ferner zur Aggregation neigt. Aggregate sind generell immunogen und werden generell durch Zugabe von Detergenzien vermieden.

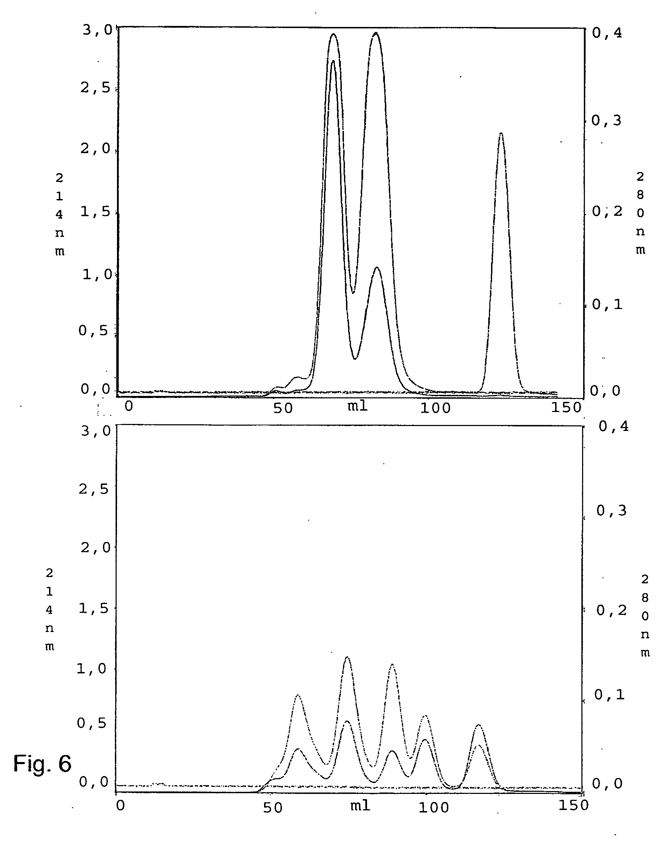

Aufgrund des hohen Anteils geladener Zuckerreste nimmt ferner der hydrodynamische Durchmesser, die Solvatisierungshülle der vorliegenden ACE2 Präparation stark zu. Dieser Umstand kann zur präparativen Aufreinigung von ACE2 über Größentrennungssäulen genutzt werden, wo das hier ausschließlich als Dimer vorliegende Protein bei einem scheinbaren Molekulargewicht von 250 kDa im Vergleich zu den berechneten 83 kDa erscheint. Ein Chromatogramm einer präparativen ACE2 Reinigung ist in Fig. 6 wiedergegeben. ACE2 (erste Abbildung) eluiert mit einer Retentionszeit von 69 Minuten. Dies entspricht einem scheinbaren Molekulargewicht zwischen dem von Thyroglobulin (erste Abbildung bei 58 Minuten, 670 kDa) und Gamma-Globulin (erste Abbildung bei 74 Minuten, 158 kDa). In diesem Bereich hoher Molekulargewichte kommen in Kulturüberständen so gut wie keine Kontaminationen vor, sodass hochreines Produkt in einem sehr einfachen Trennungsschritt abgetrennt werden kann.

### Beispiel 3: Pharmakologische Produkteigenschaften

Die erfindungsgemäße ACE2 Präparation liegt als stabile, hochreine und konzentrierte Proteinlösung in physiologischer Pufferung vor und kann ohne weitere Stabilisierung gelagert und verabreicht werden.

ACE2 ist als Monomer stabil in Lösung und zeigt aufgrund des hohen Zuckeranteils keine Aggregation. Ferner weist die ACE2 Präparation die volle enzymatische Aktivität auf.

ACE2 kann aufgrund seiner Löslichkeit als Bolus i.v. verabreicht werden. Die Bioverfügbarkeit ist aus denselben Gründen systemisch sofort nach Gabe gewährleistet.

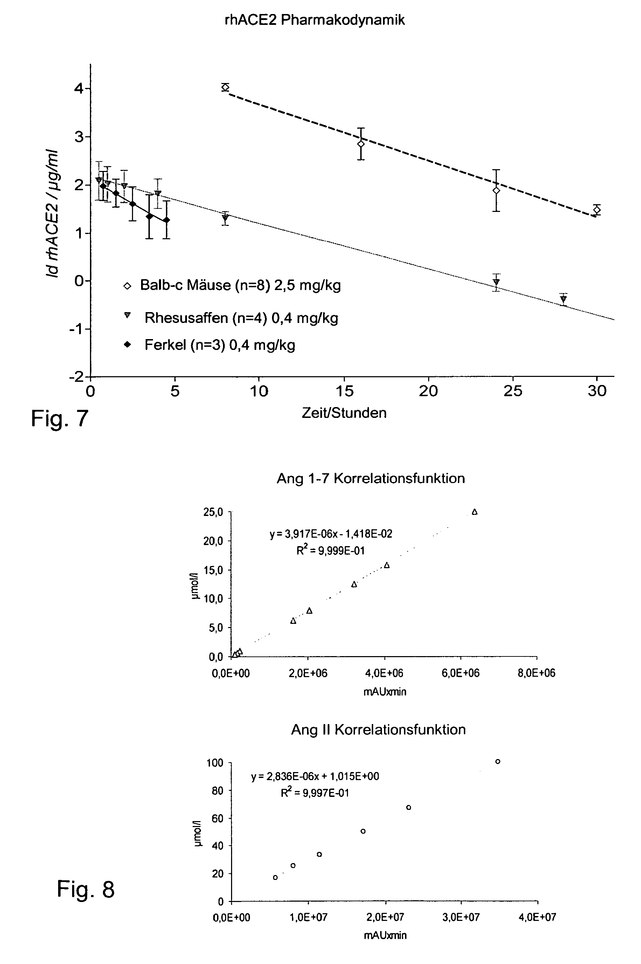

Aufgrund des hohen, stark verzweigten und komplexen Zuckeranteils wird ACE2 nur langsam abgebaut. Es resultiert daraus eine lange Halbwertszeit von mindestens 10.5 Stunden, welche in diversen Spezies, unter anderem auch in Rhesus Makaken gemessen wurde. Fig. 7 stellt die gemessene Halbwertszeit von i.v. gegebenem ACE2 in 3 Spezies dar.

Der hohe Sialinsäureanteil und das Vorliegen als Monomer bedingt ferner, dass gegen ACE2 keine neutralisierende Immunantwort aufgebaut wird. Diese wäre nicht nur für die exogene Gabe von ACE2 kontraproduktiv, sondern würde auch autologes, zellständiges ACE2 neutralisieren oder sogar ACE2 positive Zellen angreifen und lysieren.

Die beschriebene ACE2 Formulierung mitsamt sämtlicher einhergehender Produkteigenschaften ermöglicht daher erst eine effiziente Enzymsubstitutionstherapie mit rhACE2.

### Beispiel 4: Bestimmung der spezifischen ACE2 Aktivität

Die spezifische Aktivität von ACE2 Präparationen wurde durch Messung des Umsatzes von Ang II (Asp-Arg-Val-Tyr-Ile-His-Pro-Phe) bestimmt. Sämtliche Messungen wurden als Dreifachbestimmungen in einem Ansatzvolumen von 100 µl durchgeführt. Die enzymatische Reaktion wurde durch Zugabe von 250 ng/ml ACE2 zu einer 80 µM Ang II Lösung in 50 mM MES, 300 mM NaCl, 10µM ZnCl₂ und 0,01% Brij-30 bei pH 6,5 gestartet. Die Proben wurden sorgfältig gemischt und für genau 18 Minuten bei 37°C inkubiert. Die enzymatische Reaktion wurde durch Zugabe von 100 mM EDTA gestoppt. Zur Analyse wurden die Lösungen mittels RP-HPLC (Waters C18 µBondapak, 2,1x300mm, 10µm, 125Å) unter Verwendung eines linearen Gradienten von 10 bis 60% CH₃CN in 0,08% H₃PO₄ über 20 Minuten bei einer Flussrate von 1 ml/min aufgetrennt. Ferner wurden sowohl Ang II als auch Ang 1-7 Peaks in den Chromatogrammen erkannt und integriert. Die Peptidkonzentrationen wurden anhand der jeweiligen in Fig. 8 dargestellten Kalibrationskurven ermittelt. Ferner wurde der enzymatische Umsatz sowie die spezifische Enzymaktivität bestimmt.

Die Aktivität des ACE2 Produktes wurde wie zuvor beschrieben bestimmt. In Tabelle 1 sind die jeweiligen Resultate der Peakintegration sowie die berechneten Enzymdaten dargestellt.

**Tabelle 1: Enzymdaten und Reaktionsbedingungen. Es sind die Mittelwerte von Dreifachbestimmungen angegeben.**

| *Enzymatische Reaktivität* | | | | | |
|---|---|---|---|---|---|
| ***Peak Fläche*** | ***Umsatz*** | ***Reaktionszeit*** | ***ACE2 Konz.*** | ***kkat*** | ***Spezif. Aktivität*** |
| mAU.min | µmol.ml⁻¹ | min | ng.ml⁻¹ | s⁻¹ | µmol.mg⁻¹.min⁻¹ |
| **Ang II** | | | | | |
| 17149890 | 62,1 | 18 | 250 | 8,0±0,3 | 4,7±0,2 |
| **Ang 1-7** | | | | | |
| 4720141 | 23,1 | 18 | 250 | 8,8±0,2 | 5,2±0,1 |

Die ACE2 Präparation weist eine katalytische Aktivität kkat von 8,0±0,3 /s gemessen anhand des Ang II Umsatzes und 8,8±0,2 /s in Bezug auf den Ang 1-7 Umsatz auf. Beide Werte stimmen gut überein und sind deutlich höher als die Angaben von Vickers et al., (J Biol Chem. 277 (17) (2002):14838-43) der neulich eine katalytische ACE2 Aktivität von 3,5 /s veröffentlicht hat. Die Reaktionbedingungen waren identisch. Die Ursache der 240% höheren Aktivität unserer Präparation dürften post-translationelle Modifikationen und hier vor allem die N-Glykosylierung sein, die in dem Material, das Vickers verwendet hat, deutlich geringer ausgeprägt war. Sein Material wurde in Insektenzellen exprimiert und wies zwar dieselbe Amino-säuresequenz auf, war jedoch zu einem deutlich geringeren Anteil und Verzweigungsgrad glykosyliert. Es wurde ferner auch eine im Handel erhältliche ACE2 Präparation von R&D systems (cat. 933-ZN), die ebenfalls eine deutlich geringere Aktivität kkat von 2,0±0,1 /s aufwies, untersucht. Eine wesentliche Eigenschaft der erfindungsgemäße Präparation ist die erstaunlich hohe Aktivität, die vor allem durch post-translationelle Modifikationen ermöglicht wird.

### Beispiel 5: Glycoproteomische Analyse von rekombinantem ACE2

Die Probe von gereinigtem, CHO-exprimiertem ACE2 wurde auf zweierlei Arten analysiert:
Erstens wurden tryptische Peptide nach SDS-PAGE und S-Alkylierung erzeugt und mittels LC-ESI-MS (und MSMS) analysiert. Das N-terminale Peptid und mehrere interne Peptide wurden gefunden. Fünf der sieben potentiell N-glykosylierten Stellen wurden in glykosylierter Form mit Glykanstrukturen gefunden, die hauptsächlich diantennäre Glykane mit Fucose und verschiedene Mengen an Sialinsäure enthielten. Das C-terminale Peptid scheint O-glykosyliert zu sein.
Zweitens wurden freie, reduzierte N-Glykane mittels Carbon-LC-ESI-MS analysiert. Für das diantennäre, disialysierte Glykan mit Fucose konnte gezeigt werden, dass die Fucose mit dem Kern α1,6-verbunden ist und die Sialinsäure α2,3-verbunden ist. Zusätzlich zu mono- oder disialysierten, diantennären Glykanen wurde eine beträchtliche Menge an triantennärem Oligosaccharid gefunden. Vermutlich sind diese größeren Glykane hauptsächlich an jenen Glykopeptiden befestigt, die der Detektion entgingen. Eine grobe Schätzung der Masse des glykosylierten ACE2 sieht die Haupt-Glykoformen mit etwa 101-102 kDa, d.h. bestehend aus etwa 17% Kohlehydrat.
   Proteolytischer Verdau von mittels SDS-PAGE getrennten hBChE
Aliquots von ACE2 wurden einer SDS-PAGE unterzogen, entfärbt, carbamidomethyliert, mit Trypsin von Sequenzier-Qualität verdaut und aus Gel-Stücken, wie beschrieben, extrahiert. Die Extrakte wurden in einem Speed Vac-Konzentrator zur Trockene gebracht und mit Wasser, das 0,1% Ameisensäure enthielt, vor der nachfolgenden LC-MS-Analyse rekonstituiert. Für die Oligosaccharid-Analyse wurden die Peptide mit PNGase F verdaut, und die Glykane wurden unter Verwendung von C18 SPE-Spitzen gereinigt.
MS-Analyse tryptischer Peptide und Glykopeptide

Die massenspektrometrische Analyse wurde auf Q-TOF Ultima Global (Waters Micromass), ausgerüstet mit einer Standard-Elektrosprüheinheit, einem Cap-LC-System (Waters Micromass) und einem 10-Port-Lösungsmittel-Wechsel-Modul (Rheodyne), wie kürzlich beschrieben [Kolarich 2006] durchgeführt. Die Proben wurden zuerst auf einer Aquasil C18-Vorsäule (30 x 0,32 mm, Thermo Electron) unter Verwendung von Wasser als Lösungsmittel eingefangen. Die Analyse-Säule, eine Biobasic C18-Säule (100 x 0,18 mm, Thermo Electron) wurde vor dem Lösungsmittel-Wechsel auf 5% Acetonitril gehalten und danach wurde ein linearer Gradient von 5 bis 50% Acetonitril mit einer Flussrate von 2 αl/min angelegt. Alle Elutionsmittel enthielten 0,1% Ameisensäure.

Die Proben wurden auf MS- und auch auf MSMS-Art analysiert. Die Datenanalyse erfolgte mit MassLynx 4.0 SP4 software (Waters Micromass).

### Analyse freier N-Glykane von ACE2

Borhydrid-reduzierte Glykane wurden auf einer porösen graphitischen Kohlenstoff-Säule getrennt und mittels Massenspektrometrie nachgewiesen.

### Ergebnisse: Charakterisierung des Protein-Gerüsts

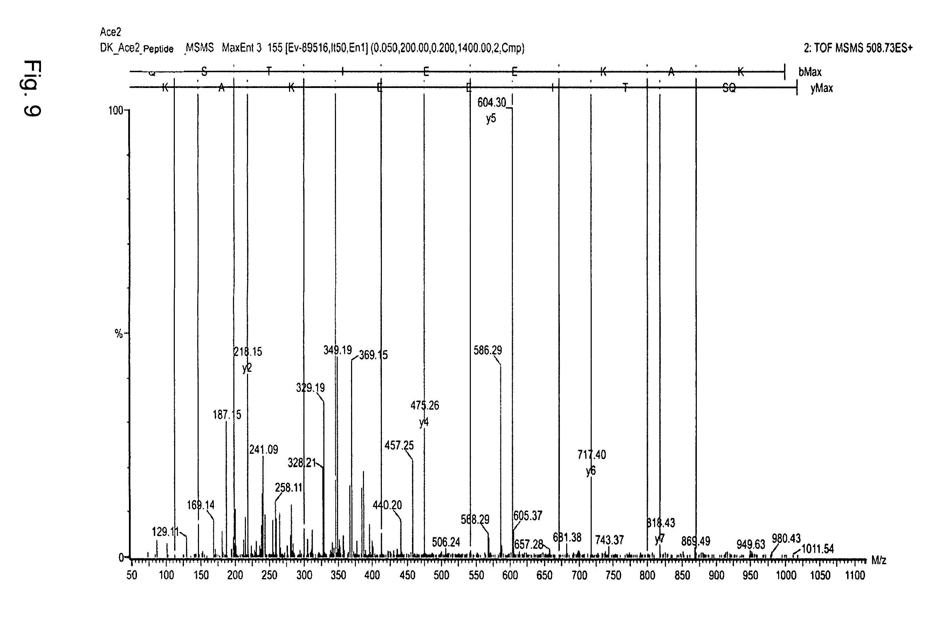

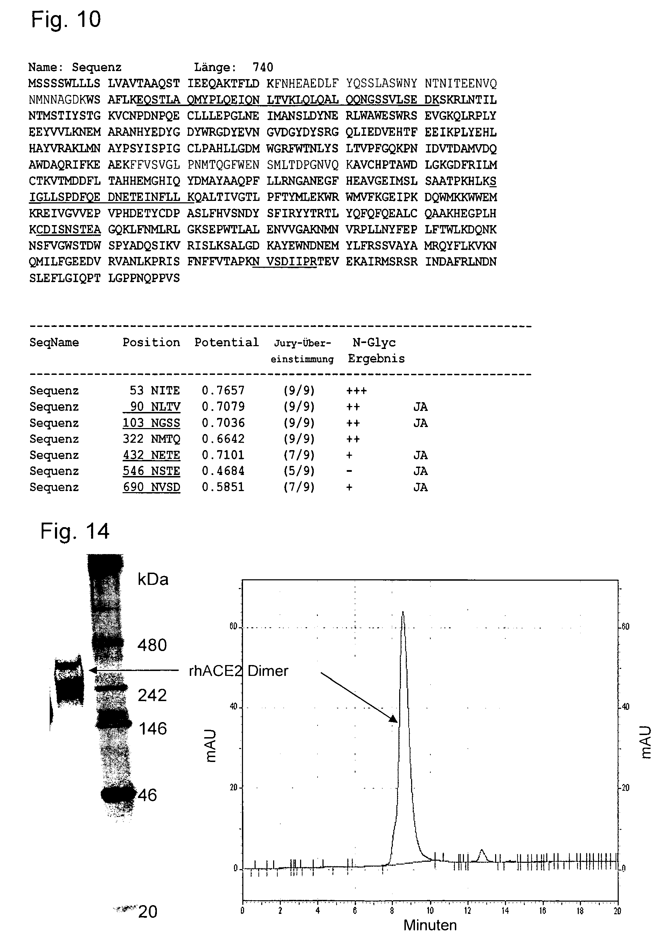

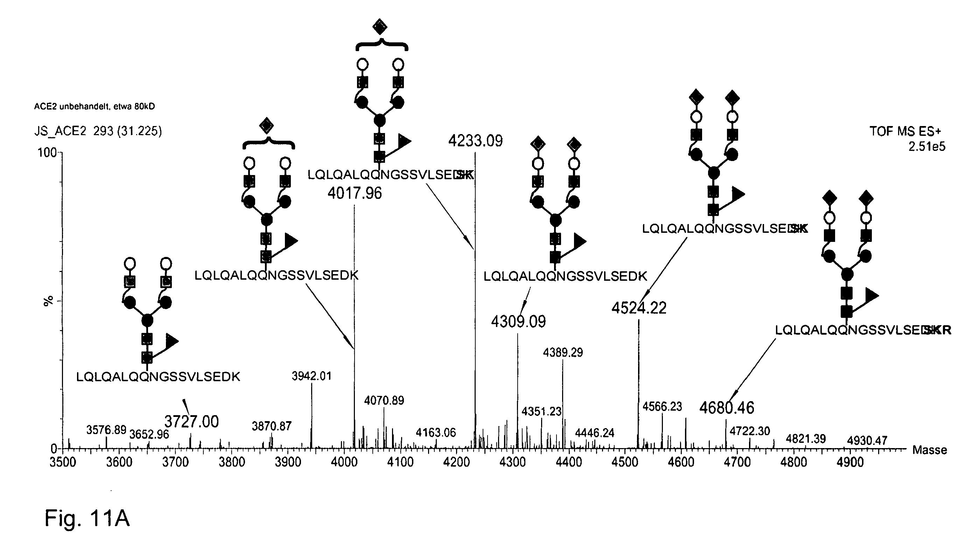

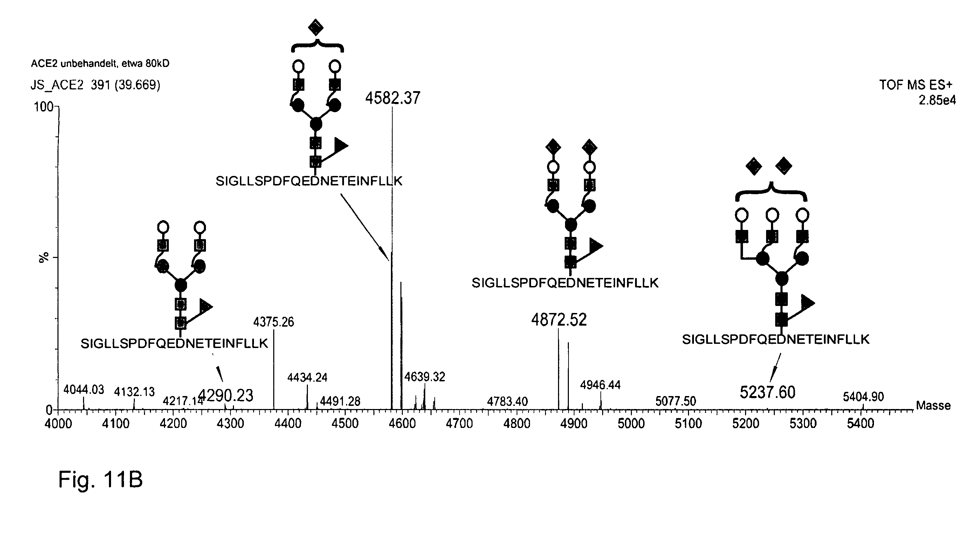

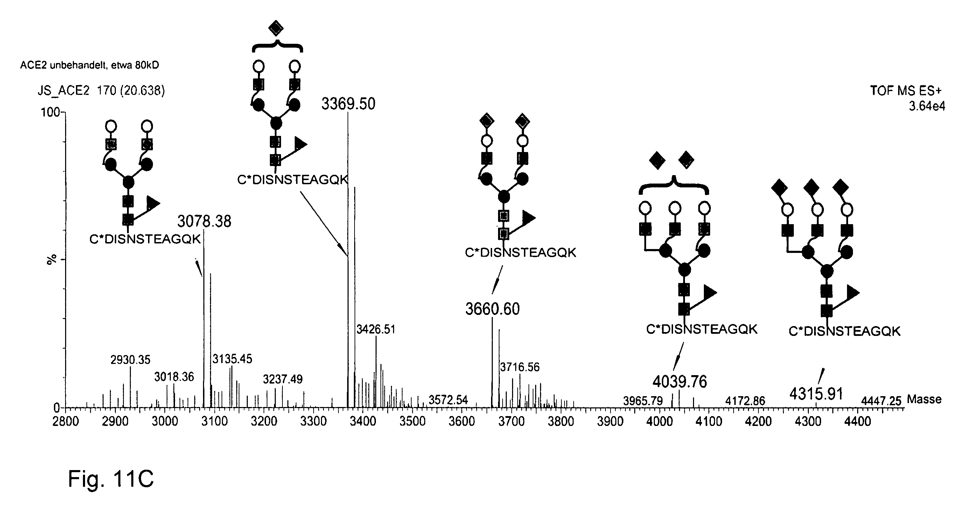

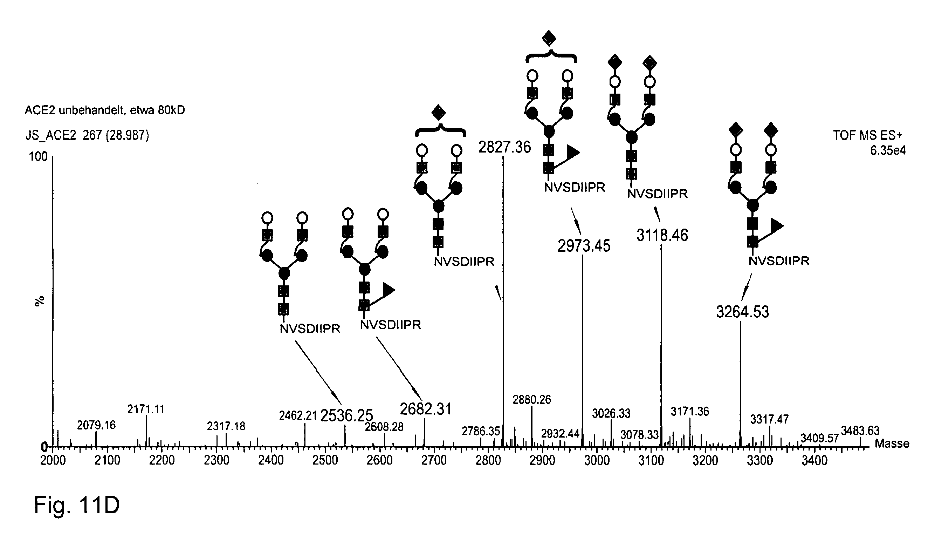

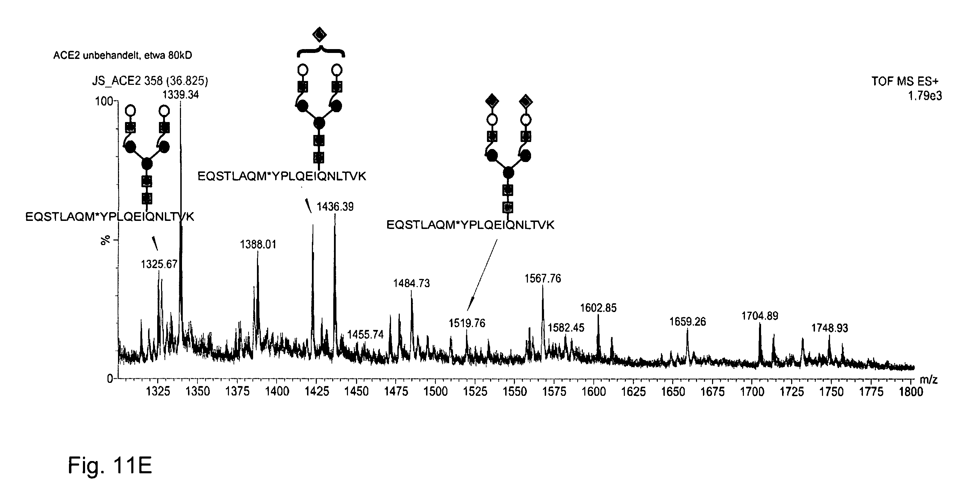

Es wurde versucht, den N-Terminus und den C-Terminus zu finden. Das Protein enthält vermutlich eine Leader-Sequenz, die die ersten 17 Aminosäuren umfasst (Heijne-Verfahren, im WWW unter psort.ims.u-tokyo.ac.jp).
Das N-terminale Peptid würde somit die Reste 18-26 / m/z = 1033,516 sein. Tatsächlich konnte die Masse 1016,46 gefunden werden, was das - sehr ungewöhnliche - Vorhandensein von Pyroglutaminsäure am N-Terminus anzeigt. Der MS/MS-Lauf bestätigte die Identität dieses Signals mit dem Peptid QSTIEEQAK (Fig. 9).
Das C-terminale Peptid konnte zuerst nicht gefunden werden. Eine Untersuchung von ACE2 mittels dem Net-O-Glyc-Programm (im WWW unter www.cbs.dtu.dk/services/NetOGlyc/) deutete auf eine hohe Wahrscheinlichkeit einer O-Glykosylierung an Ser740 hin. Tatsächlich fand die Zugabe der Massen-Inkremente für 1-2 HexNAc-Reste, 1 Hex und 1-2 Neu5Ac-Reste Signale mit dazu passenden Massen.
Von den 7 potentiell glykosylierten Stellen konnten 5 als Glykopeptide detektiert werden, die anderen überhaupt nicht - vermutlich wegen ihrer großen Größe. Bemerkenswerterweise sind die identifizierten Glykopeptide jene, von welchen vorhergesagt war, dass sie mit geringer Wahrscheinlichkeit glykosyliert sind (im WWW unter www.cbs.dtu.dk/services/NetNGlyc/). Daher könnte man vermuten, dass die übrigen XY-Stellen ebenso glykosyliert sind (Fig. 10). Fig. 11 zeigt die Massenspektren und die zugeordneten Glykan-Strukturen der N-Glykosylierungsstelle.
Die Proben-Herstellung war für die Konservierung von sialysierten Glykanen nicht optimal. Daher findet man einen bemerkenswerten Grad an Pseudo-Desialysierung, was zu Unterschieden im Vergleich zur verlässlicheren Oligosaccharid-Analyse führt (siehe unten).

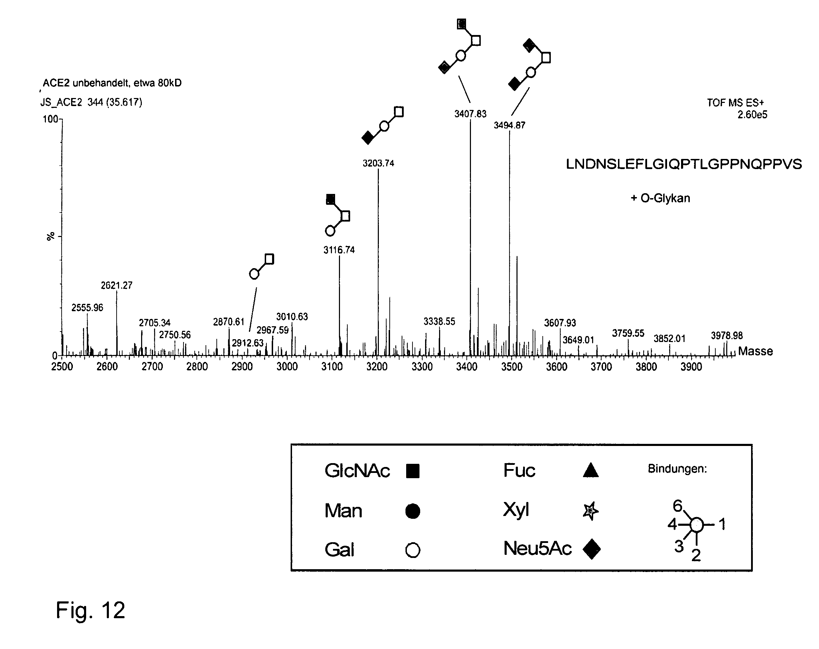

Fig. 12 zeigt das Spektrum des C-terminalen O-glykosylierten Peptids. Die strukturelle Zuordnung muss als tentativ klassifiziert werden.

### Analyse der Oligosaccharide

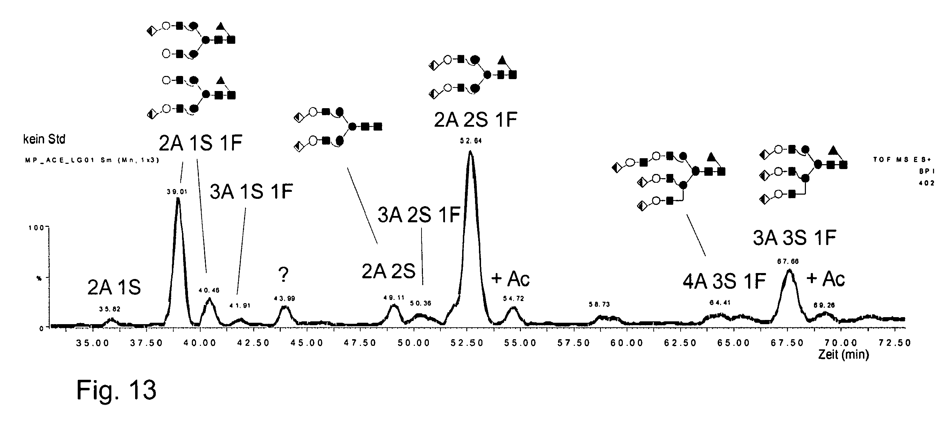

Die MS-Analyse freier Glykane zeigt das Vorhandensein von:
- diantennären N-Glykanen, hauptsächlich mit Fucose und bis zuzwei Sialinsäuren
- triantennären N-Glykanen, hauptsächlich mit Fucose und bis zu drei Sialinsäuren
- "tetraantennäre" N-Glykane mit bis zu drei Sialinsäuren, gegebenenfalls triantennär mit LacNAc-Repeats
- Spuren von tetraantennären N-Glykanen mit vier Sialinsäuren (nicht in Fig. 13 gezeigt).

Die Fucose kann mit boviner Nierenfucosidase entfernt werden, was eine Kern α1,6-Bindung der Fucose (und keine Lewis-Antigene) zeigt.

Die defucosylierten, diantennären, disialysierten N-Glykane co-eluieren mit einem bekannten Na3-4Na3-4-Standard, d.h. mit einem Glykan mit beiden Sialinsäuren in α2,3-Verbindung. Dies ist für CHO-Zellen-exprimierte Glykoproteine zu erwarten.

Zusätzlich zu triantennären, trisialysierten Glykanen mit Fucose (3A 3S F) (Fig. 13). Man bemerkt, dass die gezeigten Strukturen für die triantennären Glykane probeweise sind. Die halbvioletten/halbweißen Rhomben sind ein anderer Weg anzuzeigen, dass die Sialin-Verbindung α2,3 ist.

Triantennäre Glykane wurden nicht an Peptiden nachgewiesen. Es gibt ein Glykopeptid, welches bisher nicht gefunden wurde. Es besteht jedoch die Gefahr, dass diese Glykane ein Übertrag von der vorherigen Probe waren. Daher wurde die gesamte Herstellung und Analyse wiederholt - die triantennären Glykane wurden wiederum nachgewiesen. Es wird angenommen, dass die triantennären

Glykane (3A 3S F usw.) an jenen Stellen anhaften, für welche keine Glykopeptide gefunden werden konnten.

### Bestimmung der Molekülmasse von ACE2

| | | | |
|---|---|---|---|
| Berechnete Proteinmasse: | | 85313,7 | |
| Ohne Signalpeptid: | | 83595,7 | |
| Mit Pyroglutaminsäure: | | 83578,7 | |
| Mit 4 SS-Bindungen: | | 83570,7 | |
| 2A2SF-Inkrement: | 2352,1 | | |
| 3A3SF-Inkrement: | 3008,7 | | |
| Core1 1S Inkr.: | | 656,9 | |
| Core1 2S Inkr.: | | 947,9 | |
| Core2 1S Inkr.: | | 859,8 | |
| Mit 7 x 2A2SF-Glykanen und Core1 2S: | | | 100,984 Da |
| Mit 5 x 2A2SF +2 x 3A3SF und Core 1 2S: | | | 102,298 Da |

Das heißt (unter der Annahme, dass Stelle 53 und 322 glykosyliert sind), dass die Hauptglykoformen 100 bis 102 kDa haben werden und es auch mehrere etwas kleinere Glykoformen gibt.

### Beispiel 6: Dimerisierung von rhACE2

Nach Beispiel 1 hergestellte rACE2 wird als Dimer erhalten und in diesem Beispiel als solches analysiert. Bedingt durch die Dimerisierung von ACE2 sind sämtliche hydrophobe Proteineinheiten in das Innere des Komplexes gerichtet, wobei die geladenen Reste, wie N-gebundenen Zuckerketten nach außen ragen und die Struktur im ebenfalls geladenen physiologischen Milieu solvatisieren. Diese Dimerisierung durch Expression eines vollständig N-Glykosylierten ACE2 wurde in Gegenwart von Zn²⁺ festgestellt. Der Dimer-Komplex besteht hierbei aus 2 identischen Untereinheiten, die elektrostatisch aneinander gebunden sind und sich auch nicht mehr in physiologischen Lösungen trennen. Es kommt hierbei zur Sekretion eines Glykoproteins mit jeweils 14 stark geladenen Sialinsäurestrukturen auf jedem ACE2 Molekül sowie 28 Sialinsäurestrukturen im Dimer. Jeweils 2 Zn²⁺ Atome werden in den Komplex eingebaut und stabilisieren dessen Struktur. Die starke Ladung der Zuckerketten solvatisiert das Molekül in physiologischen wäßrigen Lösungen und zwingt die zugehörigen geladenen Proteindomänen nach außen. Der Produktionsprozeß wurde so aufgebaut, daß im Endprodukt ausschließlich ACE2 Dimere vorkommen.

Dies wird dadurch ermöglicht, dass bei Generierung des rACE2 genügend Zn²⁺-Ionen vorhanden sind (vorzugsweise kommen 1,5-5 micromolar Zn²⁺ zur Anwendung, insbesondere kann die Fermentation bei 2,5-3,5 uM Zn²⁺ durchgeführt werden) und anschließend die weiteren Behandlungsschritte in Anwesenheit von Zn²⁺-Ionen vorgenommen werden.

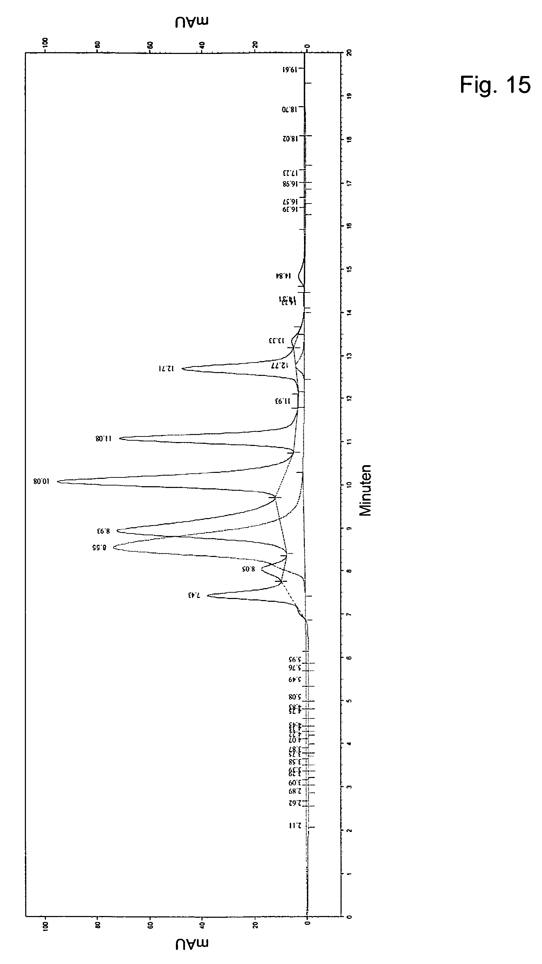

In Figur 14 und 15 wurde die Dimerisierung des ACE2 Komplexes mit unterschiedlichen Methoden nachgewiesen. In der nativen Poly-Acryllamid-Gel-Elektrophorese wurde das Protein nach Silberfärbung als einzelne Bande einer Größe von ca. 250 kDa nachgewiesen. Mittels Größentrennungschromatographie auf einer Zorbax G-450 Säule in Gegenwart von 10% Acetonitril in 220 mM Na-Phosphatpuffer bei pH 7,0 erscheint das Produkt ebenfalls als einzelner Peak bei einer einem Molekulargewicht von ca. 250 kDa entsprechenden Retentionszeit. Bemerkenswert ist in beiden Fällen, daß ausschließlich dimerisiertes Protein nachgewiesen werden kann. Weder Monomerstrukturen, noch höhermolekulare Aggregate konnten nachgewiesen werden.

## Patentansprüche

1. Rekombinantes ACE2 Polypeptid, **dadurch gekennzeichnet, dass** es glykosiliert ist, wobei die Glycogruppen eines ACE2 Polypeptid Monomers in Summe mindestens 14 Sialylsäure-Reste aufweisen und das ACE2 Polypetid als Dimer vorliegt.

2. Rekombinantes ACE2 Polypeptid nach Anspruch 1, **dadurch gekennzeichnet, dass** es an mindestens 80% der möglichen N-Glykosylierungspositionen glykosyliert ist und einen Zuckeranteil von größer als 10% (Massen-%, bezogen auf das gesamte ACE2-Molekül), vorzugsweise größer als 15%, insbesondere größer als 20 %, aufweist.

3. Rekombinantes ACE2 Polypeptid nach Anspruch 1 oder 2, **dadurch gekennzeichnet, dass** mindestens 70% der glykosylierten N-Glykosylierungspositionen eine Struktur unabhängig voneinander ausgewählt aus den Formeln 1-8: 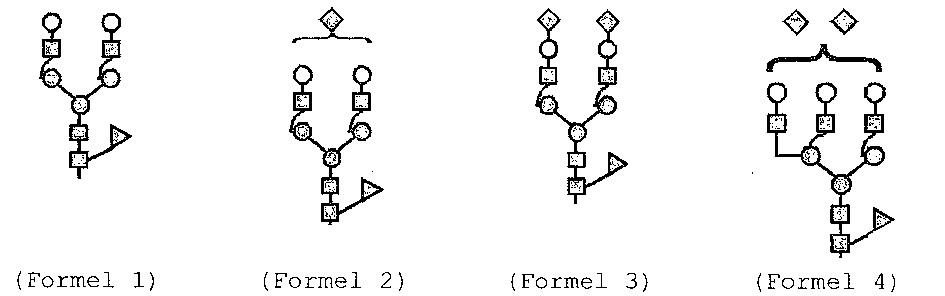 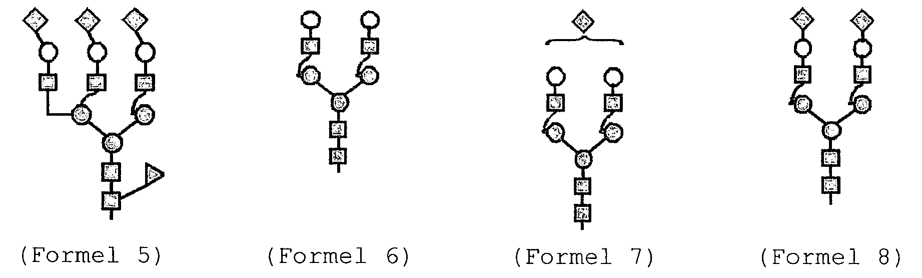 wobei
G lc N A c ▭
M a n ○
G a l ○
F u c △
X y l 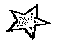
N e u 5 A c 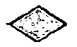
ist, aufweisen.

4. Rekombinantes ACE2 Polypeptid nach einem der Ansprüche 1 bis 3, **dadurch gekennzeichnet, dass** mindestens 80%, vorzugsweise mindestens 90%, insbesondere 100%, der glykosylierten N-Glykosylierungspositionen eine Struktur der Formeln 1-8 aufweisen.

5. Rekombinantes ACE2 Polypeptid nach einem der Ansprüche 1 bis 4, **dadurch gekennzeichnet, dass** das ACE2 Monomer ein Molekulargewicht von mindestens 101 kDa, vorzugsweise mindestens 105 kDa, besonders bevorzugt mindestens 109 kDa, insbesondere mindestens 113 kDa, speziell bevorzugt mindestens 117 kDa, am meisten bevorzugt mindestens 119 kDa, aufweist.

6. Rekombinantes ACE2 Polypeptid nach einem der Ansprüche 1 bis 5, **dadurch gekennzeichnet, dass** ein zu Ser740 der SEQ ID NO: 1 korrespondierendes Serin O-glykosyliert ist.

7. Rekombinantes ACE2 Polypeptid nach einem der Ansprüche 1 bis 6, **dadurch gekennzeichnet, dass** mind. 70%, vorzugsweise mind. 80%, insbesondere mind. 90%, am meisten bevorzugt 100% der glykosylierten N-Glykosylierungsstellen Sialinsäure aufweisen, vorzugsweise sind die N-Glykosylierungsstellen entsprechend Asn53, Asn90, Asn103, Asn322, Asn432, Asn546, Asn690 der SEQ ID NO: 1 sialysiert.

8. Rekombinantes ACE2 Polypeptid nach einem der Ansprüche 1 bis 7, **dadurch gekennzeichnet, dass** mind. 30%, vorzugsweise mind. 40%, insbesondere mind. 55%, am meisten bevorzugt mind. 70% der glykosylierten N-Glykosylierungsstellen mindestens zwei Sialinsäuren aufweisen.

9. Präparation von rekombinanten ACE2 Polypeptiden, umfassend ein Polypeptid nach einem der Ansprüche 1 bis 8, **dadurch gekennzeichnet, dass** der Anteil von ACE2 Polypeptiden mit einem Molekulargewicht von unter 100 kDa, bevorzugt von unter 104 kDa, speziell bevorzugt von unter 108 kDa, insbesondere von unter 112 kDa, besonders bevorzugt von unter 117 kDa, am meisten bevorzugt von unter 119 kDa, unter 20%, vorzugsweise unter 10%, insbesondere bevorzugt unter 5%, am meisten bevorzugt unter 1%, im speziellen bei 0%, liegt.

10. Präparation nach Anspruch 9, **dadurch gekennzeichnet dass** der Anteil von ACE2 Multimeren unter 20%, vorzugsweise unter 10%, insbesondere bevorzugt unter 5%, am meisten bevorzugt unter 1%, im speziellen bei 0%, liegt.

11. Präparation nach einem der Ansprüche 9 bis 10, **dadurch gekennzeichnet, dass** der Anteil an ACE2 Dimeren an ACE2 Molekülen mindestens 70%, vorzugsweise mindestens 80%, besonders bevorzugt mindestens 90% im speziellen bevorzugt mindestens 95% am meisten bevorzugt mindestens 99%, ist.

12. Verfahren zur Herstellung von rekombinanten ACE2 Polypeptiden oder einer Präparation von rekombinantem ACE2, vorzugsweise nach einem der Ansprüche 1 bis 11, umfassend die Schritte der Einbringung eines ACE2 codierenden Polynukleotides, vorzugsweise eines ACE2 ohne Transmembrandomäne codierenden Polynukleotides, in eukaryontischen Zellen, Expression des ACE2 Polypeptids und Sammeln des exprimierten ACE2, insbesondere in dimerer Form, wobei vorzugsweise bei der Expression die Zn^{z+}-Konzentration mindestens 1,5 micromolar ist.

13. Rekombinantes ACE2 Polypeptid oder Präparation von rekombinanten ACE2 Polypeptiden erhältlich durch ein Verfahren der vorhergehenden Ansprüche.

14. Stabil mit einem ACE2 codierenden Polynukleotid transfizierte eukaryontische Zellinie, vorzugsweise CHO Zelllinie, die ACE2 nach einem der Ansprüche 1 bis 11 exprimiert.

15. Zelle nach Anspruch 14, die eine ACE2-Produktivität von mindestens 10 pg/Zelle/Tag, vorzugsweise mindestens 15 pg/Zelle/Tag, insbesondere bevorzugt von mindestens 20 pg/Zelle/Tag aufweist.
